# EUROPÄISCHE PATENTSCHRIFT

(11) **EP 2 001 972 B1**
(45) Veröffentlichungstag und Bekanntmachung des Hinweises auf die Patenterteilung: **04.05.2011**
(21) Anmeldenummer: 07711763.8
(22) Anmeldetag: 03.03.2007
(51) Int. Cl.: C09K 11/06, H05B 33/14, C07C 209/10, C07C 211/55

(54) **MATERIALEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 01.04.2006 DE 102006015183
(43) Veröffentlichungstag der Anmeldung: 17.12.2008
(73) Patentinhaber: Merck Patent GmbH, 64293 Darmstadt (DE)
(72) Erfinder: STOESSEL, Philipp, 60487 Frankfurt am Main (DE); HEIL, Holger, 64295 Darmstadt (DE); BUESING, Arne, 65959 Frankfurt am Main (DE)
(86) Internationale Anmeldenummer: PCT/EP2007/001839
(87) Internationale Veröffentlichungsnummer: WO 2007/115610

(56) Entgegenhaltungen:
- EP-A2- 0 786 926
- WO-A-2006/000388

## Beschreibung

Die vorliegende Erfindung betrifft Arylamine, deren Verwendung zur Herstellung organischer Elektrolumineszenzvorrichtungen und organische Elektrolumineszenzvorrichtungen enthaltend diese Verbindungen.

Der allgemeine Aufbau organischer Elektrolumineszenzvorrichtungen, die halbleitende organische Verbindungen enthalten und zur Emission von Licht im sichtbaren Spektralbereich befähigt sind, ist beispielsweise in US 4539507, US 5151629, EP 0676461 und WO 98/27136 beschrieben. Allerdings zeigen diese Vorrichtungen noch erhebliche Probleme, die für die Verwendung in hochwertigen Displays einer dringenden Verbesserung bedürfen. So ist insbesondere die operative Lebensdauer bei blauer Emission noch nicht ausreichend, so dass bis dato nur einfache Anwendungen kommerziell realisiert werden konnten. Auch sind die üblicherweise verwendeten blauen Emitter thermisch nicht ausreichend stabil.

In JP 04-184892 werden Distilbenamine, Tristilbenamine und weitere Stilbenderivate als emittierende Verbindungen für OLEDs beschrieben. Die Vorrichtungen mit den vorgeschlagenen Verbindungen weisen keine zufrieden stellenden Lebensdauern auf. Außerdem ist die thermische Stabilität dieser Verbindungen bei der Device-Herstellung durch Vakuum-Sublimation nicht ausreichend. Die Zersetzungsprodukte führen zu einer Verunreinigung der Elektrolumineszenzvorrichtung und damit zu schlechteren elektronischen Eigenschaften. Auch andere Stilbenamine, wie sie gemäß dem Stand der Technik verwendet werden (z. B. gemäß JP 08-239655 oder EP 1167488) weisen nur eine unzureichende thermische Stabilität auf.

In WO 06/000388 werden Aryl-substituierte Tristilbenamine als emittierende Verbindungen für OLEDs beschrieben. Die Vorrichtungen mit den vorgeschlagenen Verbindungen zeigen hellblaue, aber keine tiefblaue Emission.

In EP 0786926 A2 werden Diarylamino-substituierte 9,10-Diphenylanthracenderivate als blaue Emitter für die Verwendung in der emittierenden Schicht einer organischen Elektrolumineszenzvorrichtung offenbart.

Allgemein wird bei Stilbenaminen beobachtet, dass diese bei hoher Temperatur, wie sie bei der Sublimation zur Reinigung der Materialien und bei der Deviceherstellung verwendet wird, unerwünschte Nebenreaktionen zeigen (Schema 1). So zeigen diese Verbindungen eine thermisch induzierte cis-trans-Isomerisierung. Das cis-Stilbenamin kann in einer intramolekularen Ringschlussreaktion zum entsprechenden Dihydrophenanthren und in Anwesenheit eines Oxidationsmittels, beispielsweise Resten von Sauerstoff, zum entsprechenden Phenanthren weiterreagieren. Dies führt zu uneinheitlichen Materialmischungen in der OLED, was eine reproduzierbare Deviceherstellung erschwert. Weiterhin beobachtet man generell eine thermisch induzierte Olefinmetathesereaktion. Diese führt einerseits zu niedermolekularen Verbindungen, welche zu Verunreinigungen der OLED führen, und andererseits zu einem hochmolekularen verharzten Rückstand, da die Metathesereaktion bei mehreren Stilbengruppen im Molekül zu einer Vernetzung des Materials führt. Dieser verharzte Rückstand führt bei vielen Materialien zu einem erheblichen Materialverlust. Zusätzlich führt höhermolekulares aufgedampftes Material, das durch Olefinmetathese entstanden ist, zu einer längerwelligen Emission, da diese Verbindungen ein weiter ausgedehntes π-Elektronensystem aufweisen.

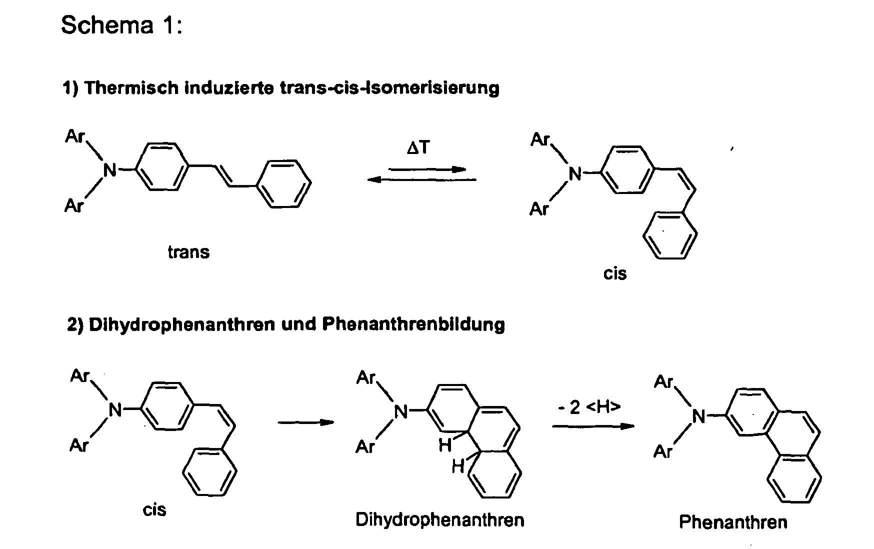

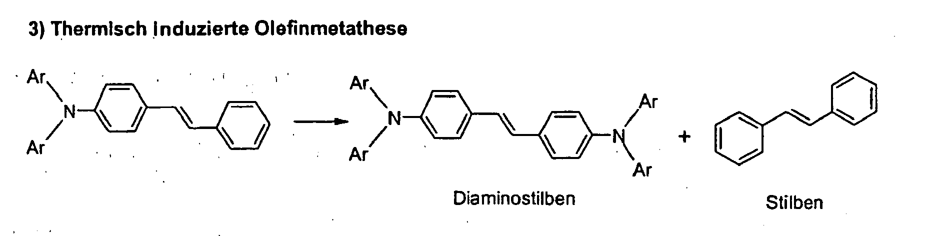

Es war daher Aufgabe der vorliegenden Erfindung, hierfür Verbesserungen anzubieten, insbesondere Verbindungen mit verbesserter Lebensdauer bei verbesserten tiefblauen Farbkoordinaten und hoher thermischer Stabilität.

Überraschend wurde gefunden, dass organische Elektrolumineszenzvorrichtungen, die Triarylamin-Derivate, welche mit Dibenzosuberen, Dibenzooxepin, Dibenzoazepin oder Derivaten dieser Verbindungen substituiert sind, in der emittierenden Schicht enthalten, deutliche Verbesserungen gegenüber dem Stand der Technik aufweisen. Mit diesen Materialien werden verbesserte Lebensdauern bei tiefblauer Emissionsfarbe und guter Effizienz erhalten. Weiterhin weisen diese Materialien eine höhere thermische Stabilität auf als die Stilbenamine, die gemäß dem Stand der Technik als blau emittierende Verbindungen verwendet werden. Diese Verbindungen und deren Verwendung in OLEDs, insbesondere in der emittierenden Schicht, sind daher der Gegenstand der vorliegenden Erfindung.

Gegenstand der Erfindung sind Verbindungen der Formel (1), 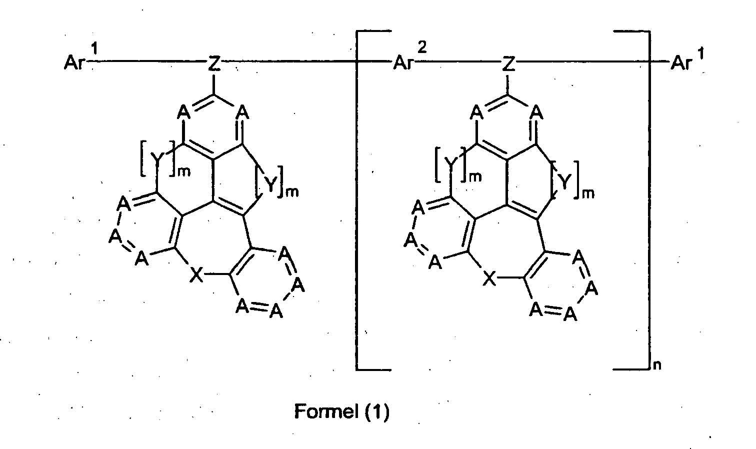 wobei für die verwendeten Symbole und Indizes gilt:
- Z: ist gleich oder verschieden bei jedem Auftreten N, P, As oder P=O;
- X, Y: ist gleich oder verschieden bei jedem Auftreten CR₂, C=O, O, S, NR, SiR₂, PR, P(=O)R, S(=O) oder SO₂;
- A: ist gleich oder verschieden bei jedem Auftreten CR oder N;
- Ar¹: ist gleich oder verschieden bei jedem Auftreten ein monovalentes aromatisches oder heteroaromatisches Ringsystem mit 5 bis 40 aromatischen Ringatomen, welches mit einem oder mehreren Resten R substituiert sind kann;
- Ar²: ist gleich oder verschieden bei jedem Auftreten ein bivalentes aromatisches oder heteroaromatisches Ringsystem mit 5 bis 40 aromatischen Ringatomen, welches mit einem oder mehreren Resten R substituiert sind kann;
- R: ist gleich oder verschieden, bei jedem Auftreten H, F, Cl, Br, I, CN, NO₂, Si(R¹)₃, N(R¹)₂, B(OR¹)₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R¹ substituiert sein kann, wobei eine oder mehrere nicht benachbarte CH₂-Gruppen durch -R¹C=CR¹-, -C≡C-, Si(R¹)₂, Ge(R¹)₂, Sn(R¹)₂, C=O, C=S, C=Se, C=NR¹, -O-, -S-, -N(R¹)- oder -CONR¹- ersetzt sein können und wobei ein oder mehrere H-Atome durch F, Cl, Br, I, CN oder NO₂ ersetzt sein können, oder ein aromatisches oder hetero- aromatisches Ringsystem mit 5 bis 40 aromatischen Ringatomen, das mit einem oder mehreren Resten R¹ substituiert sein kann, oder eine Aryloxy- oder Heteroaryloxygruppe mit 5 bis 24 aromatischen Ringatomen, die durch einen oder mehrere Reste R¹ substituiert sein kann, oder eine Kombination aus zwei, drei, vier oder fünf dieser Systeme; dabei können auch zwei oder mehrere Substituenten R miteinander ein mono- oder polycyclisches, aliphatisches oder aromatisches Ringsystem bilden;
- R¹: ist bei jedem Auftreten gleich oder verschieden H oder ein Kohlenwasserstoffrest mit 1 bis 20 C-Atomen, welcher aliphatisch oder aromatisch oder eine Kombination aus aliphatisch und aromatisch sein kann; dabei können auch ein oder mehrere H-Atome durch F ersetzt sein; dabei können auch zwei oder mehrere Reste R¹ miteinander ein mono- oder polycyclisches, aliphatisches oder aromatisches Ringsystem bilden;
- n: ist 0, 1, 2 oder 3;
- m: ist gleich oder verschieden bei jedem Auftreten 0 oder 1, wobei m = 0 bedeutet, dass die Gruppe Y nicht vorhanden ist und stattdessen in diesen Positionen Reste R gebunden sind.

Unter einer Arylgruppe bzw. einer Heteroarylgruppe im Sinne dieser Erfindung wird eine aromatische Gruppe bzw. heteroaromatische Gruppe mit einem gemeinsamen aromatischen π-Elektronensystem verstanden. Dies kann im Sinne dieser Erfindung ein einfacher Homo- oder Heterocyclus sein, beispielsweise Benzol, Pyridin, Thiophen, etc., oder es kann ein kondensiertes aromatisches Ringsystem sein, in dem mindestens zwei aromatische oder heteroaromatische Ringe, beispielsweise Benzolringe, miteinander "verschmolzen", d. h. durch Anellierung einander ankondensiert sind, also mindestens eine gemeinsame Kante und dadurch auch ein gemeinsames aromatisches π-Elektronensystem aufweisen. Diese Aryl- oder Heteroarylgruppen können substituiert oder unsubstituiert sein; ebenso können gegebenenfalls vorhandene Substituenten weitere Ringsysteme bilden. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen darstellen, da es sich hierbei um separate aromatische π-Elektronensysteme handelt.

Ein aromatisches Ringsystem im Sinne dieser Erfindung enthält 6 bis 30 C-Atome im Ringsystem. Ein heteroaromatisches Ringsystem im Sinne dieser Erfindung enthält 2 bis 30 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können oder in dem eine oder mehrere Aryl- oder Heteroarylgruppen an eine nicht-aromatische cyclische Gruppe kondensiert sind. So sollen beispielsweise mehrere miteinander verknüpfte Aromaten, wie z. B. Biphenyl, oder auch Systeme wie 9,9'-Spirobifluoren, 9,9-Diarylfluoren, Triarylamin, Diarylether, etc. als aromatische Ringsysteme im Sinne dieser Erfindung verstanden werden. Ebenso werden Systeme wie Dibenzosuberen, Dibenzooxepin, Dibenzoazepin, etc. als aromatische Ringsysteme im Sinne dieser Erfindung verstanden.

Unter einer cyclischen Alkylgruppe im Sinne dieser Erfindung werden sowohl monocyclische wie auch bi- und polycyclische Alkylgruppen verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Adamantyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 bis 40 aromatischen Ringatomen, welches noch jeweils mit den oben genannten Resten R bzw. R¹ substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Tetracen, Pentacen, Benzpyren, Biphenyl, Biphenylen, Terphenyl, Terphenylen, Fluoren, Spirobifluoren, Truxen, Isotruxen, Dihydrophenanthren, Dihydropyren, Tetrahydropyren, cis- oder trans-Indenofluoren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4 Thiadiazol, 1,3,5-Triazin, 1,2,4-Triazin, 1,2,3-triazin, Tetrazol, 1,2,4,5-Tetrazin, 1,2,3,4-Tetrazin, 1,2,3,5-Tetrazin, Purin, Pteridin, Indolizin, Benzothiadiazol, Triphenylamin, Naphthyldiphenylamin, Dinaphthylphenylamin, Diphenylether, Dibenzosuberen, Dibenzooxepin, Dibenzoazepin und Kombinationen dieser Systeme.

Im Folgenden werden bevorzugte Ausführungsformen für Verbindungen der Formel (1) beschrieben.

In einer bevorzugten Ausführungsform der Erfindung steht das Symbol Z, gleich oder verschieden bei jedem Auftreten, für N oder P=O. In einer besonders bevorzugten Ausführungsform der Erfindung steht das Symbol Z für N.

In einer bevorzugten Ausführungsform der Erfindung steht maximal ein Symbol A in jedem aromatischen Ring für N, und die anderen Symbole A in diesem aromatischen Ring stehen für CR. In einer besonders bevorzugten Ausführungsform der Erfindung steht das Symbol A für CR.

In einer bevorzugten Ausführungsform der Erfindung steht mindestens eine Gruppe Ar¹ für eine Gruppe der Formel (2), 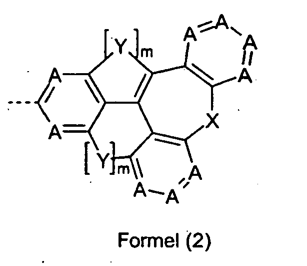 wobei die Symbole und Indizes dieselbe Bedeutung haben, wie oben beschrieben, und die gestrichelte Bindung die Anknüpfung an die Gruppe Z bedeutet. Dabei steht bevorzugt maximal ein Symbol A in jedem aromatischen Ring für N und die anderen Symbole A in diesem aromatischen Ring stehen für CR; besonders bevorzugt steht das Symbol A für CR.

Besonders bevorzugt sind Verbindungen der Formel (1a), 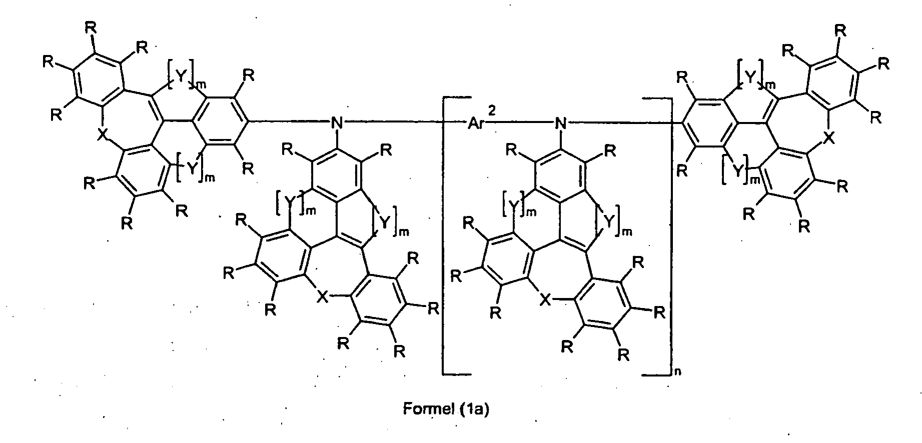 wobei die Symbole und Indizes dieselbe Bedeutung haben, wie oben beschrieben.

Bevorzugtsind weiterhin Verbindungen der Formel (1) bzw. (1a), in denen das Symbol X, gleich oder verschieden bei jedem Auftreten, für CR₂, O, NR oder SiR₂ steht. Besonders bevorzugt sind Verbindungen der Formel (1) bzw. (1a), in denen das Symbol X, gleich oder verschieden bei jedem Auftreten, für CR₂ oder O steht.

Wenn das Symbol X für eine Gruppe der Formel CR₂ steht, dann steht R in der Einheit CR₂ bevorzugt, gleich oder verschieden bei jedem Auftreten, für eine geradkettige Alkylgruppe mit 1 bis 4 C-Atomen oder eine verzweigte Alkylgruppe mit 3 oder 4 C-Atomen, wobei jeweils ein oder mehrere H-Atome durch F ersetzt sein können, oder für eine Aryl- oder Heteroarylgruppe mit 5 bis 10 aromatischen Ringatomen, oder eine Kombination aus zwei oder drei dieser Systeme; dabei können auch die zwei Reste R der Gruppe CR₂ miteinander ein mono- oder polycyclisches, aliphatisches oder aromatisches Ringsystem bilden und dadurch ein Spirosystem aufbauen. Besonders bevorzugte Reste R sind Methyl, tert-Butyl, Phenyl, ortho-Tolyl, para-Tolyl oder para-tert-Butylphenyl. Dabei können jeweils zwei Phenylgruppen auch miteinander ein Ringsystem bilden und dadurch ein Spirosystem aufspannen, wie in Formel (3) am Grundgerüst des Dibenzosuberens gezeigt:

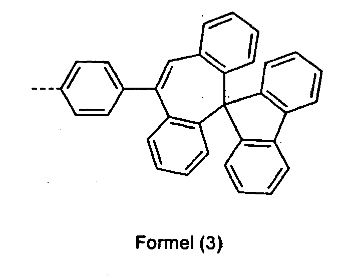

Bevorzugt sind weiterhin Verbindungen der Formel (1) bzw. (1a), in denen das Symbol Y, gleich oder verschieden bei jedem Auftreten, für CR₂, O, NR oder SiR₂ steht. Besonders bevorzugt sind Verbindungen der Formel (1) bzw. (1a), in denen das Symbol Y, gleich oder verschieden bei jedem Auftreten, für CR₂ oder O steht.

Bevorzugt sind weiterhin Verbindungen der Formel (1) bzw. (1a), in denen das Symbol R, welches nicht an der Gruppe X gebunden ist, gleich oder verschieden bei jedem Auftreten, für H, F, Si(R¹)₃, B(OR¹)₂, eine geradkettige Alkyl- oder Alkoxygruppe mit 1 bis 6 C-Atomen oder eine verzweigte oder cyclische Alkyl- oder Alkoxygruppe mit 3 bis 10 C-Atomen, wobei jeweils eine oder mehrere CH₂-Gruppen durch -R¹C=CR¹-, Si(R¹)₂, -O-, -S- oder -N(R²)- ersetzt sein können und wobei jeweils ein oder mehrere H-Atome durch F ersetzt sein können, oder für eine Aryl- oder Heteroarylgruppe mit 5 bis 14 aromatischen Ringatomen, oder eine Kombination aus zwei oder drei dieser Systeme steht; dabei können auch zwei oder mehrere benachbarte Reste R miteinander ein mono- oder polycyclisches, aliphatisches oder aromatisches Ringsystem bilden. Besonders bevorzugte Reste R sind ausgewählt aus der Gruppe bestehend aus H, F, Si(R¹)₃, B(OR¹)₂, geradkettigen Alkylgruppen mit 1 bis 4 C-Atomen, verzweigten Alkylgruppen mit 3 bis 5 C-Atomen oder cyclischen Alkylgruppen mit 5 bis 10 C-Atomen, wobei jeweils ein oder mehrere H-Atome durch F ersetzt sein können, oder monovalenten Aryl- oder Heteroarylgruppen mit 6 bis 10 aromatischen Ringatomen oder eine Kombination aus zwei dieser Systeme.

Bevorzugt sind weiterhin Verbindungen gemäß Formel (1 b), 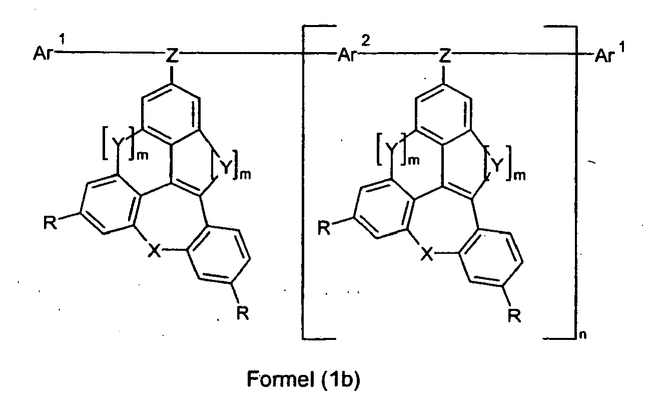 wobei die Symbole und Indizes dieselbe Bedeutung haben, wie oben beschrieben.

Bevorzugt sind weiterhin Verbindungen der Formel (1) bzw. (1a) bzw. (1 b), in denen das Symbol Ar², gleich oder verschieden bei jedem Auftreten, für ein aromatisches oder heteroaromatisches Ringsystem mit 6 bis 24 aromatischen Ringatomen steht. Besonders bevorzugt steht das Symbol Ar², gleich oder verschieden bei jedem Auftreten, für eine Aryl-, Heteroaryl- oder Biarylgruppe mit 6 bis 16 aromatischen Ringatomen oder für ein durch R substituiertes oder unsubstituiertes Fluoren, Spirobifluoren oder Indenofluoren.

Bevorzugt sind weiterhin Verbindungen der Formel (1) bzw. (1a) bzw. (1 b), in denen der Index n für 0, 1 oder 2, besonders bevorzugt für 0 oder 1, ganz besonders bevorzugt für 0 steht.

Bevorzugt sind weiterhin Verbindungen der Formel (1) bzw. (1 a) bzw. (1 b), in denen an jeder Einheit der Formel (2) höchstens ein Index m gleich 1 ist. Besonders bevorzugt sind Verbindungen der Formel (1) bzw. (1a), in denen alle Indizes m gleich 0 sind.

Besonders bevorzugt sind also Verbindungen der Formel (1 c), 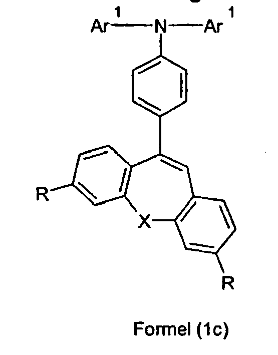 wobei die Symbole dieselbe Bedeutung haben, wie oben beschrieben, und worin die Symbole Ar¹ bevorzugt eine Gruppe der oben abgebildeten Formel (2) darstellen.

Bevorzugt sind weiterhin Verbindungen der Formel (1) bzw. (1a) bis (1 c), in denen alle Gruppen X gleich gewählt sind und alle Gruppen Y, falls vorhanden, gleich gewählt sind und in denen die Gruppen auch jeweils gleich substituiert sind, also symmetrische Verbindungen. Insbesondere bevorzugt sind für n = 0 Verbindungen, die eine dreizählige Symmetrieachse aufweisen.

Beispiele für bevorzugte Verbindungen der Formel (1) bzw. (1 a) bis (1 c) sind die im Folgenden abgebildeten Verbindungen (1) bis (33).

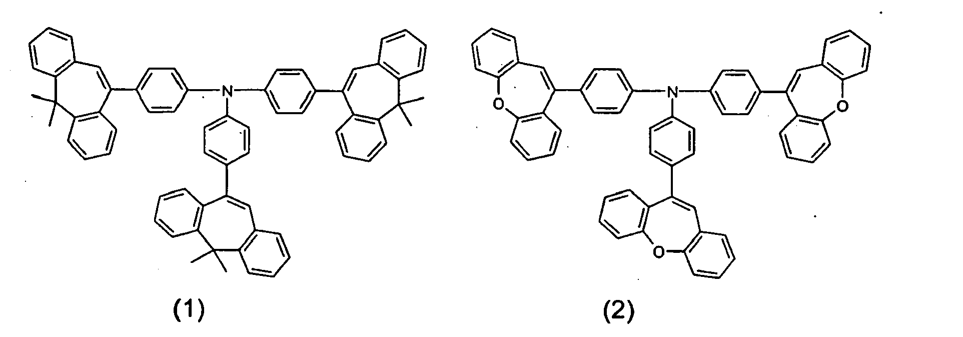

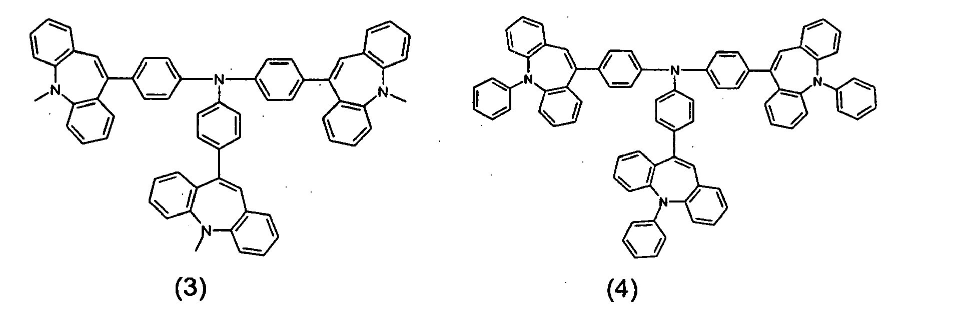

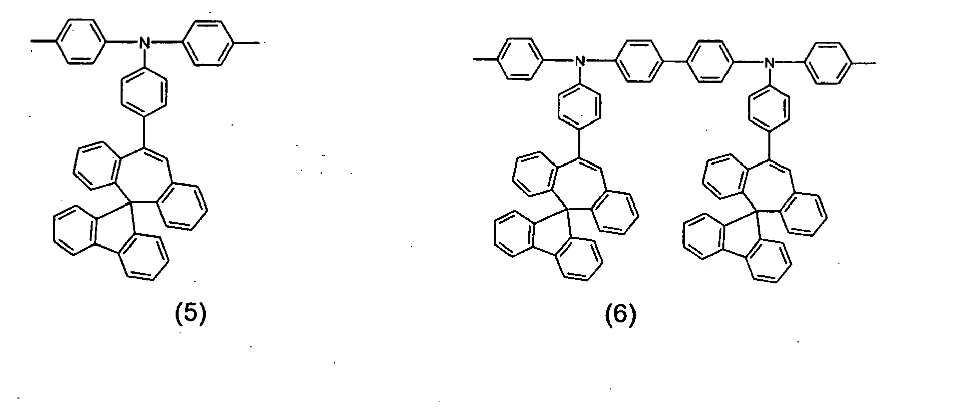

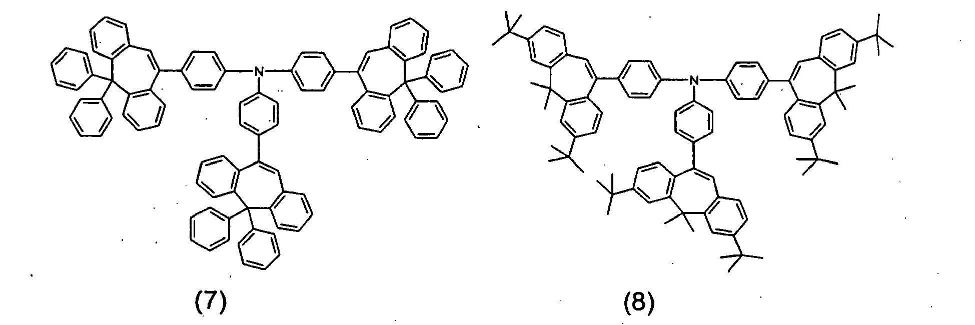

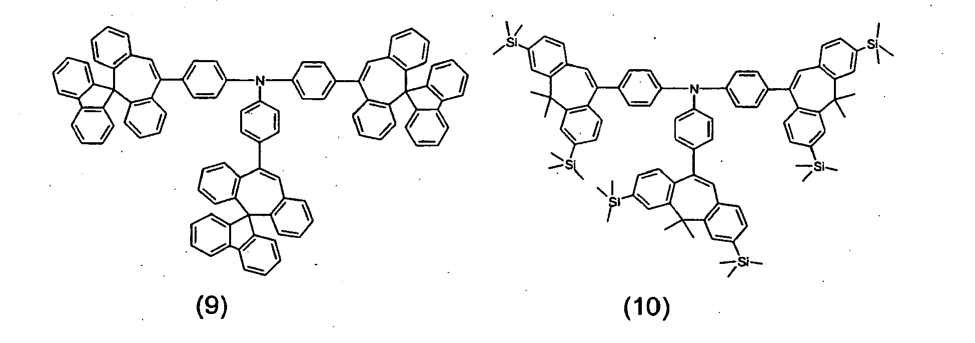

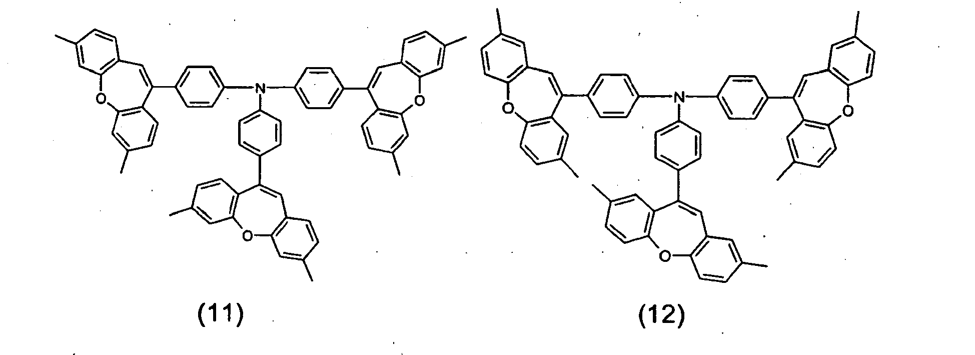

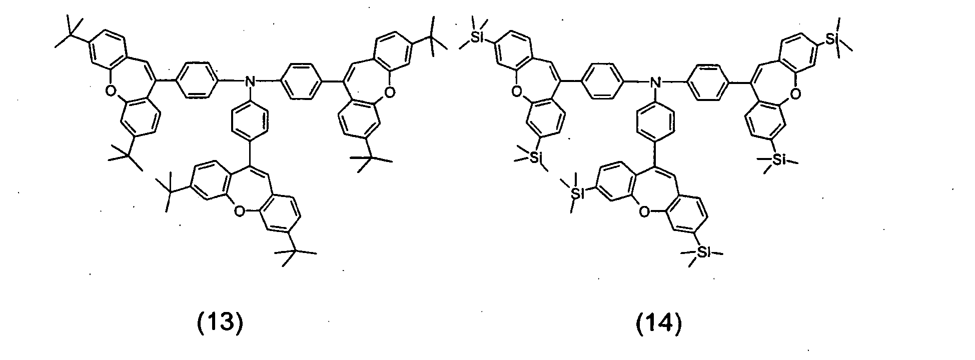

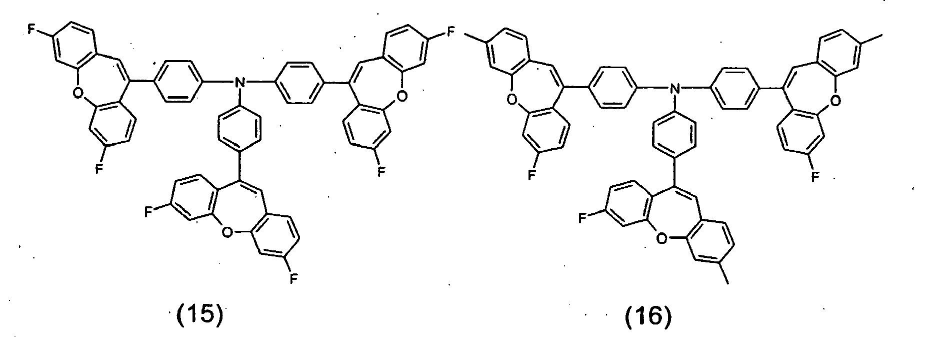

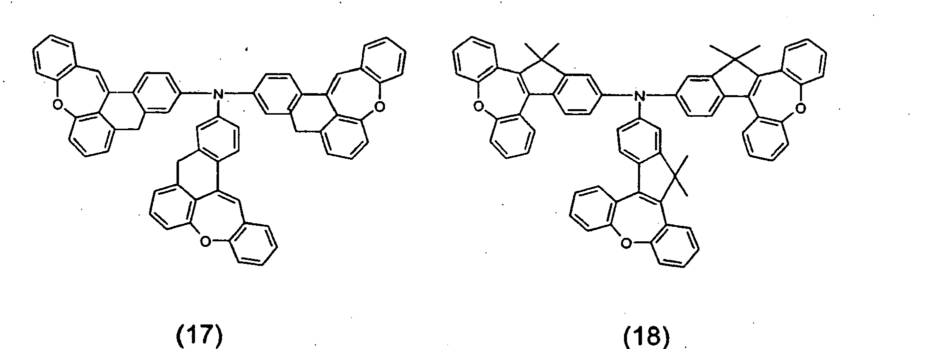

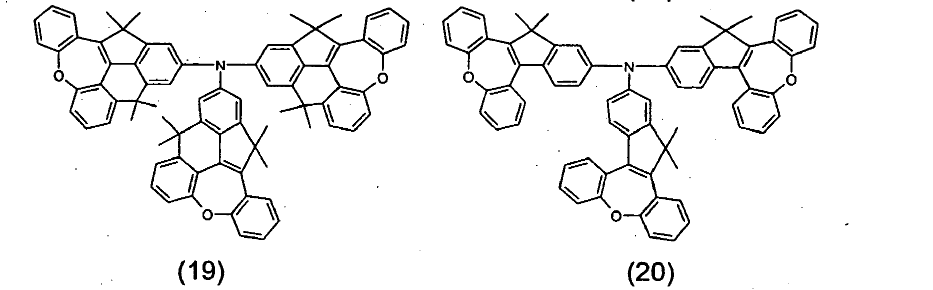

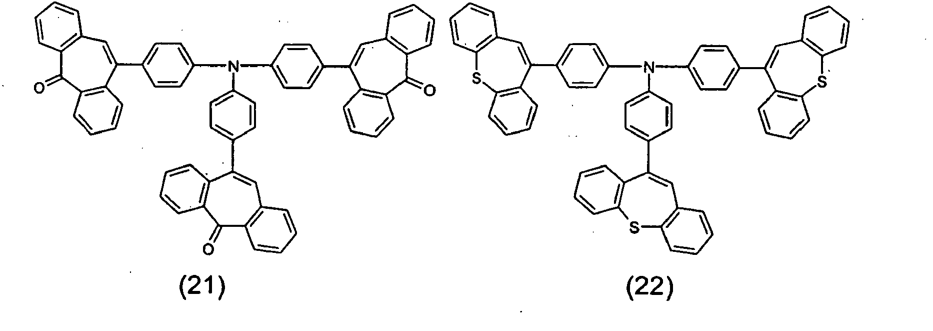

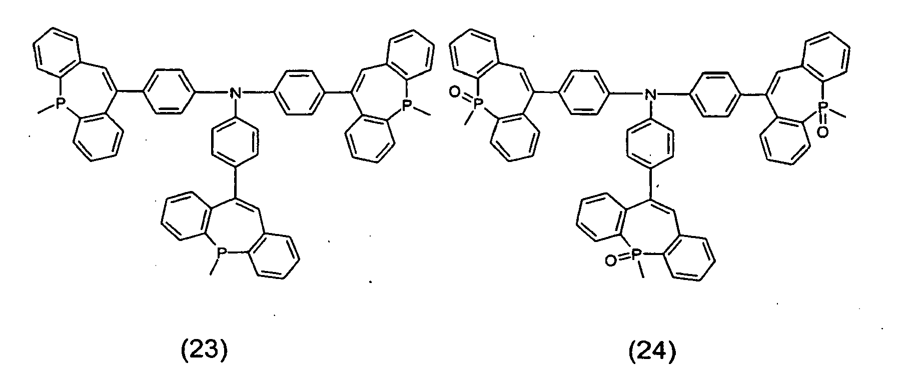

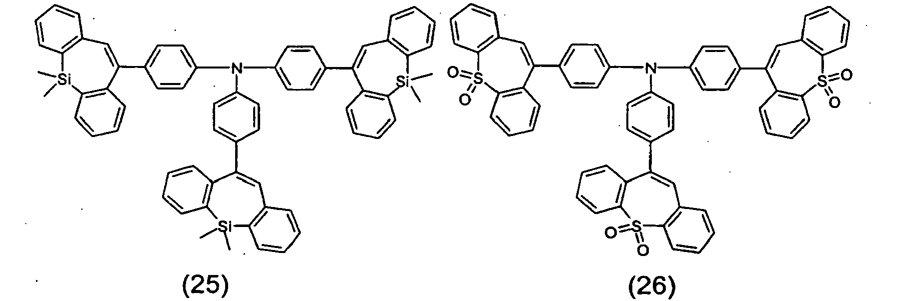

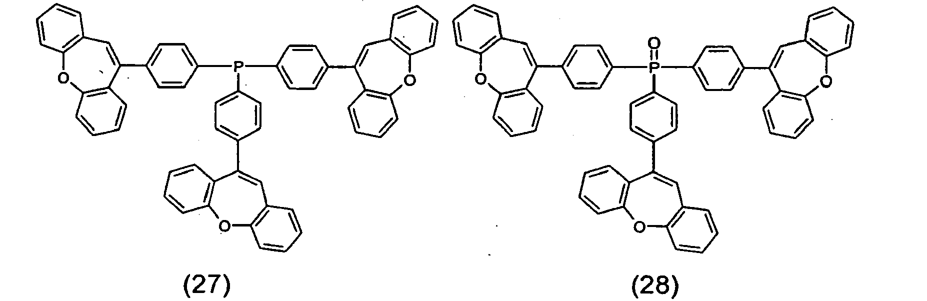

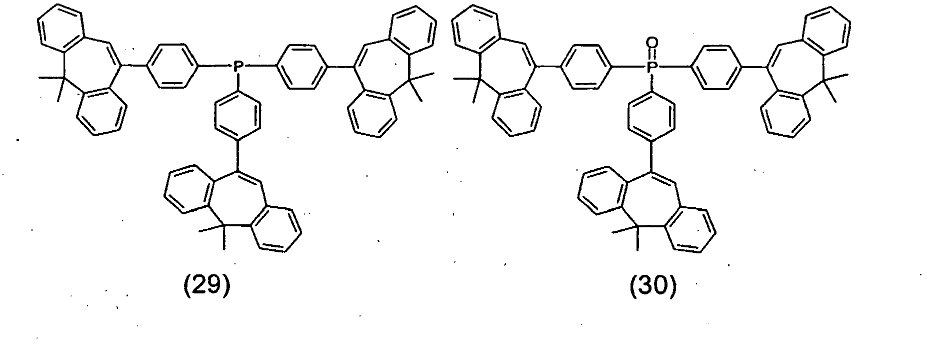

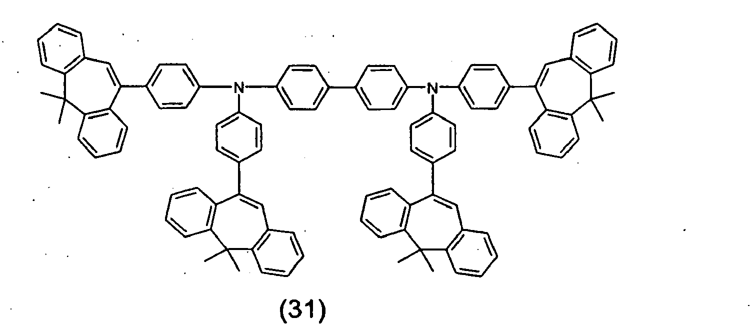

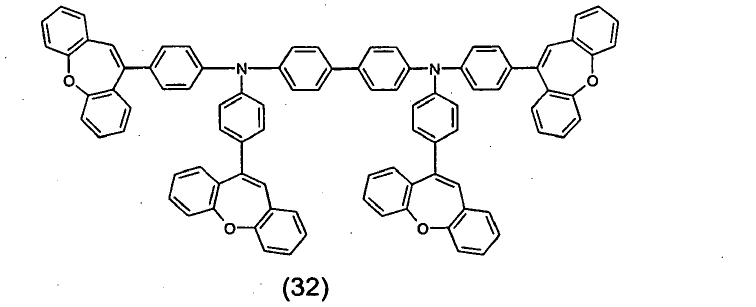

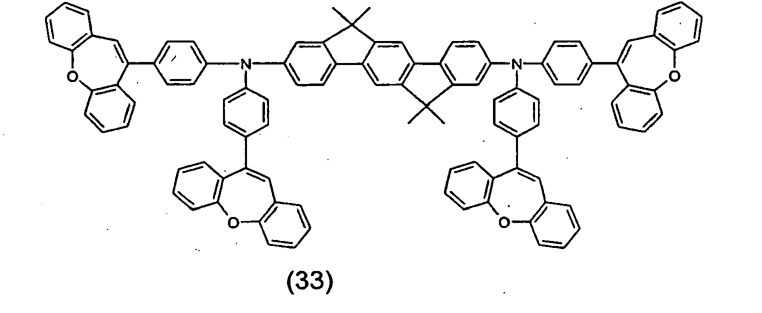

Die oben beschriebenen erfindungsgemäßen Verbindungen können beispielsweise auch als Comonomere zur Erzeugung entsprechender konjugierter, teilkonjugierter oder nicht-konjugierter Polymere, Oligomere oder auch als Kern von Dendrimeren Verwendung finden. Hierfür eignen sich insbesondere halogenierte Verbindungen, wobei die Polymerisation dann bevorzugt über die Halogenfunktionalität erfolgt.

Weiterer Gegenstand der Erfindung sind somit konjugierte, teilkonjugierte und nicht-konjugierte Polymere, Oligomere oder Dendrimere enthaltend eine oder mehrere Verbindungen gemäß Formel (1), wobei ein oder mehrere Reste R Bindungen der Verbindung gemäß Formel (1) zum Polymer oder Dendrimer darstellen.

Diese Polymere können weitere Wiederholeinheiten enthalten. Diese weiteren Wiederholeinheiten sind vorzugsweise ausgewählt aus der Gruppe, bestehend aus Fluorenen (z. B. gemäß EP 842208 oder WO 00/22026), Spirobifluorenen (z. B. gemäß EP 707020, EP 894107 oder WO 06/061181), Triarylaminen, Para-phenylenen (z. B. gemäß WO 92/18552), Carbazolen (z. B. gemäß WO 04/070772 und WO 04/113468), Thiophenen (z. B. gemäß EP 1028136), Dihydrophenanthrenen (z. B. gemäß WO 05/014689), Indenofluorenen (z. B. gemäß WO 04/041901 und WO 04/113412), aromatischen Ketonen (z. B. gemäß WO 05/040302), Phenanthrenen (z. B. gemäß WO 05/104264) und/oder Metallkomplexen, insbesondere ortho-metallierten Iridiumkomplexen. Dabei sei ausdrücklich darauf hingewiesen, dass die Polymere auch mehrere verschiedene Wiederholeinheiten aufweisen können, welche aus einer oder mehreren der oben genannten Gruppen ausgewählt sind.

Die Synthese der Verbindungen erfolgt nach Methoden der organischen Chemie, die dem Fachmann geläufig sind. So können beispielsweise Dibenzosuberen (Schmuck et al., Synthesis 2002, 5, 655), 5,5'-Dimethyldibenzosuberen (Vinatoru et al., Org. Prep. Proced. Int. 1975, 7(2), 98), Dibenzooxepin (Hess et al., J. Am. Chem. Soc. 1967, 89(11), 2746) und N-Methyldibenzoazepin (Ohta et al., Chem. Pharm Bull. 1981, 29(5), 1221) und entsprechende substituierte Derivate nach Literaturmethoden synthetisiert werden. Unzählige weitere Derivate der o. g. Grundkörper sind in der Literatur beschrieben worden und spielen vor allem als Pharmazwischenprodukte eine Rolle. Diese Verbindungen können dann beispielsweise in einer Heck-Kupplung mit einem Triarylamin, welches an mindestens einer Arylgruppe durch Chlor, Brom, Iod oder eine andere Abgangsgruppe wie beispielsweise Sulfonat substituiert ist, beispielsweise mit Tris(para-bromphenyl)amin, umgesetzt werden (Schema 2).

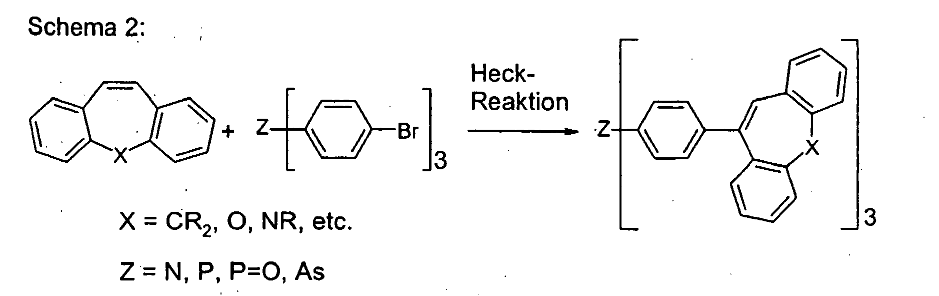

Alternativ ist eine Brom-Adition an die Doppelbindung, gefolgt von einer HBr-Eliminierung, anschließender Umwandlung der Vinylbromids in die entsprechende Boronsäure und abschließende Suzuki-Kupplung möglich (Schema 3). Dabei wird die Boronsäure bevorzugt nicht isoliert, sondern wird in situ hergestellt und direkt für die Suzuki-Kupplung eingesetzt. Als Ligand für das Palladium eignet sich bei der Suzuki-Kupplung hier besonders Tris(1-furyl)phosphin.

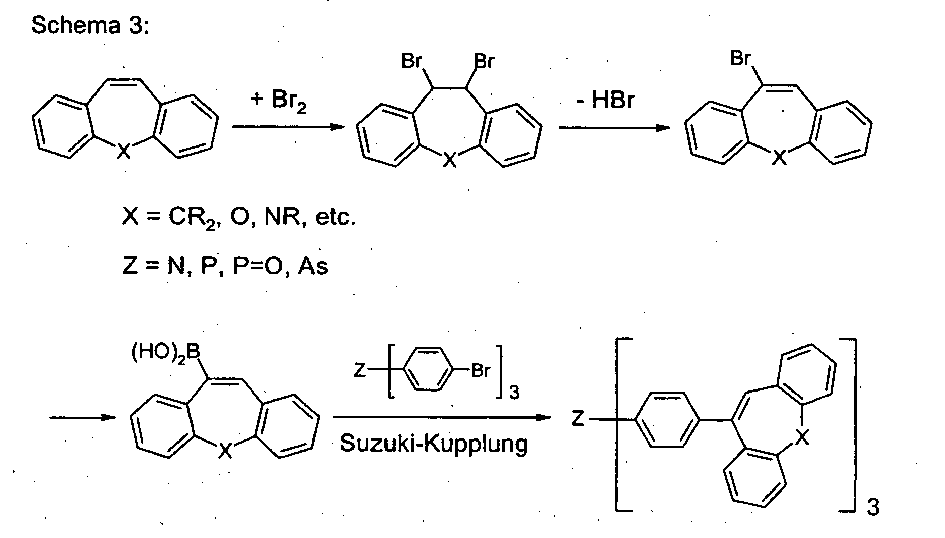

Ein weiterer Gegenstand der vorliegenden Erfindung ist daher ein Verfahren zur Herstellung von Verbindungen gemäß Formel (1), dadurch gekennzeichnet, dass ein Boronsäurederivat gemäß Formel (4), 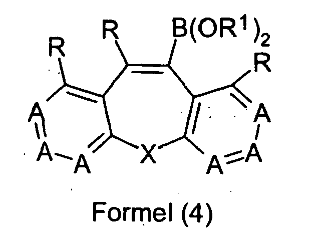 wobei die Symbole die oben aufgeführte Bedeutung haben, in einer Suzuki-Kupplung mit der zentralen Einheit der Verbindung gemäß Formel (1) umgesetzt wird, wobei diese Chlor, Brom, Iod, Triflat, Tosylat oder OSO₂R¹ als reaktive Gruppe enthält, wobei R¹ dieselbe Bedeutung hat, wie oben beschrieben.

Die Verbindungen gemäß Formel (1) können in organischen elektronischen Vorrichtungen verwendet werden, insbesondere in organischen Elektrolumineszenzvorrichtungen.

Gegenstand der Erfindung ist daher weiterhin die Verwendung von Verbindungen gemäß Formel (1) in organischen elektronischen Vorrichtungen, insbesondere in organischen Elektrolumirieszenzvorrichtungen.

Gegenstand der Erfindung sind weiterhin organische elektronische Vorrichtungen, enthaltend mindestens eine organische Schicht, dadurch gekennzeichnet, dass die organische Schicht mindestens eine Verbindung der Formel (1) enthält.

Die organische elektronische Vorrichtung ist bevorzugt ausgewählt aus organischen Elektrolumineszenzvorrichtungen (OLEDs, PLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), organischen Photorezeptoren, lichtemittierenden elektrochemischen Zellen (LECs) oder organischen Laserdioden (O-Laser). Besonders bevorzugt sind organische Elektrolumineszenzvorrichtungen (OLEDs, PLEDs).

Die organische Elektrolumineszenzvorrichtung enthält Anode, Kathode und mindestens eine emittierende Schicht. Außer diesen Schichten kann sie weitere Schichten enthalten, insbesondere ausgewählt aus Lochinjektionsschicht, Lochtransportschicht, Lochblockierschicht, Elektronentransportschicht, Elektroneninjektionsschicht und/oder Charge-Generation Layer (T. Matsumoto et al., Multiphoton Organic EL Device Having Charge Generation Layer, IDMC 2003, Taiwan; Session 21 OLED (5)). Es muss nicht notwendigerweise jede dieser Schichten vorhanden sein. Als Lochinjektions- und Lochtransportmaterialien eignen sich beispielsweise aromatische Amine, wie sie üblicherweise gemäß dem Stand der Technik verwendet werden, welche auch p-dotiert sein können. Als Elektronentransportmaterialien eignen sich beispielsweise Metallchelatkomplexe, z. B. AlQ₃, Verbindungen auf Basis elektronenarmer Heterocyclen, z. B. Triazinderivate, oder Verbindungen enthaltend aromatische Carbonyle oder Phosphinoxide, wie z. B. beschrieben in WO 05/084081 und WO 05/084082, welche jeweils auch n-dotiert sein können. Als Elektroneninjektionsmaterialien eignen sich insbesondere Fluoride und Oxide der Alkali- und Erdalkalimetalle, beispielsweise NaF, BaF₂, CaF₂, LiF oder Li₂O.

Die Verbindungen gemäß Formel (1) können in der organischen elektronischen Vorrichtung in verschiedenen Funktionen verwendet werden.

In einer bevorzugten Ausführungsform der Erfindung wird die Verbindung gemäß Formel (1) als emittierende Verbindung in einer emittierenden Schicht verwendet. Die Verbindungen gemäß Formel (1) sind insbesondere dann für diese Funktion besonders geeignet, wenn das Symbol Z für N steht und die Symbole X und, falls vorhanden, Y für CR₂, O, NR und/oder SiR₂.

Die Verbindungen werden bevorzugt als Mischung mit einem Hostmaterial verwendet. Unter einem Hostmaterial wird in einem System aus Host und Dotand (binäres System) diejenige Komponente verstanden, die in dem System im höheren Anteil vorliegt. Bei einem System aus einem Host und mehreren Dotanden (ternäre und höhere Systeme) wird als Host diejenige Komponente verstanden, deren Anteil der höchste in der Mischung ist. Als Hostmaterialien kommen verschiedene Stoffklassen in Frage, wie sie üblicherweise gemäß dem Stand der Technik als Hostmaterialien für fluoreszierende organische Elektrolumineszenzvorrichtungen verwendet werden. Bevorzugte Hostmaterialien sind ausgewählt aus den Klassen der Oligo-arylene (z. B. 2,2',7,7'-Tetraphenyl-spirobifluoren gemäß EP 676461 oder Dinaphthylanthracen), insbesondere der Oligo-arylene enthaltend kondensierte aromatische Gruppen, der Oligo-arylenvinylene (z. B. DPVBi (Bis-diphenylvinylbiphenyl) oder Spiro-DPVBi gemäß EP 676461), der polypodalen Metallkomplexe (z. B. gemäß WO 04/081017), der lochleitenden Verbindungen (z. B. gemäß WO 04/058911), insbesondere der Triarylamin-Derivate und der Carbazol-Derivate, der elektronenleitenden Verbindungen, insbesondere Ketone, Phosphinoxide und Sulfoxide (z. B. gemäß WO 05/084081 und WO 05/084082), der Atropisomere (z. B. gemäß WO 06/048268), der Ansa-Verbindungen (z. B. gemäß WO 06/097208), der Cycloalkylphenylanthracene (z. B. gemäß der nicht offen gelegten Anmeldung DE 102005026651.7) oder der Boronsäurederivate (z. B. gemäß WO 06/177052). Besonders bevorzugte Hostmaterialien sind ausgewählt aus den Klassen der Oligoarylene, enthaltend Naphthalin, Anthracen, Pyren und/oder Perylen oder Atropisomeren dieser Verbindungen, der Ketone, der Phosphinoxide, der Sulfoxide und der Boronsäurederivate. Ganz besonders bevorzugte Hostmaterialien sind ausgewählt aus den Klassen der Oligoarylene, enthaltend Anthracen und/oder Pyren oder Atropisomeren dieser Verbindungen und der Phosphinoxide.

Der Anteil der Verbindung gemäß Formel (1) in der Mischung beträgt zwischen 0.1 und 99.0 Gew.-%, bevorzugt zwischen 0.5 und 50.0 Gew.-%, besonders bevorzugt zwischen 1.0 und 20.0 Gew.-%, insbesondere zwischen 1.0 und 10.0 Gew.-%. Entsprechend beträgt der Anteil des Hostmaterials in der Mischung zwischen 1.0 und 99.9 Gew.-%, bevorzugt zwischen 50.0 und 99.5 Gew.-%, besonders bevorzugt zwischen 80.0 und 99.0 Gew.-%, insbesondere zwischen 90.0 und 99.0 Gew.-%.

In einer weiteren Ausführungsform der Erfindung weist die organische Elektrolumineszenzvorrichtung mehrere emittierende Schichten auf, wobei mindestens eine dieser Schichten mindestens eine Verbindung gemäß Formel (1) enthält, bevorzugt in Kombination mit einem Hostmaterial. Besonders bevorzugt weisen diese Emissionsschichten insgesamt mehrere Emissionsmaxima zwischen 380 nm und 750 nm auf, so dass insgesamt weiße Emission resultiert, d. h. in der bzw. den weiteren emittierenden Schichten wird noch mindestens eine weitere emittierende Verbindung verwendet, die fluoreszieren oder phosphoreszieren kann und die gelbes, orange oder rotes Licht emittiert, Insbesondere bevorzugt sind Dreischichtsysteme, wobei mindestens eine dieser Schichten mindestens eine Verbindung gemäß Formel (1) enthält, bevorzugt in Kombination mit einem Hostmaterial und wobei die drei Schichten blaue, grüne und orange oder rote Emission zeigen (für den prinzipiellen Aufbau siehe z. B. WO 05/011013).

In einer weiteren Ausführungsform der Erfindung wird die Verbindung gemäß Formel (1) als Lochtransport- und/oder Lochinjektionsmaterial verwendet. Dies gilt insbesondere dann, wenn das Symbol Z für N oder P, insbesondere für N, steht und die Symbole X und, wenn vorhanden, Y für CR₂, O, NR und/oder SiR₂ stehen. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Wenn die Verbindung gemäß Formel (1) als Lochtransport- bzw. als Lochinjektionsmaterial verwendet wird, kann es bevorzugt sein, wenn sie mit Elektronenakzeptor-Verbindungen dotiert ist, beispielsweise mit F₄-TCNQ oder mit Verbindungen, wie sie in EP 1476881 oder EP 1596445 beschrieben werden. Wird die Verbindung gemäß Formel (1) als Lochtransport- und/oder Lochinjektionsmaterial in einer Lochtransportschicht und/oder einer Lochinjektionsschicht eingesetzt, kann auch ein Anteil von 100 % bevorzugt sein, also die Verwendung dieser Verbindung als Reinmaterial.

Es ist weiterhin bevorzugt, die Verbindungen der Formel (1) als Elektronentransportmaterial und/oder als Lochblockiermaterial für fluoreszierende und phosphoreszierende OLEDs und/oder als Triplett-Matrixmaterial für phosphoreszierende OLEDs einzusetzen. Dies gilt insbesondere für Verbindungen, in denen die Gruppe Z für P=O steht und/oder die Gruppen X und, falls vorhanden, Y für C=O, P=O, S=O oder SO₂ stehen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Hohe Löslichkeit lässt sich durch geeignete Substitution der Verbindungen erreichen.

Weiterer Gegenstand sind Mischungen enthaltend mindestens eine Verbindung gemäß Formel (1) und mindestens ein Hostmaterial.

Die erfindungsgemäßen organischen Elektrolumineszenzvorrichtungen weisen folgenden überraschenden Vorteil gegenüber dem Stand der Technik auf:
1. Die Stabilität der Vorrichtungen wird höher im Vergleich zu Systemen gemäß dem Stand der Technik, was sich vor allem in einer deutlich höheren Lebensdauer zeigt.
2. Die Verbindungen weisen eine höhere thermische Stabilität auf als Stilbenamine, die gemäß dem Stand der Technik als blaue Emitter verwendet werden. Insbesondere wird bei diesen Verbindungen weder eine thermisch induzierte cis-trans-Isomerisierung noch eine thermisch induzierte Metathesereaktion beobachtet. So lassen sich diese Verbindungen nahezu verlustfrei und ohne im Device zu Verunreinigungen zu führen, sublimieren und ermöglichen eine reproduzierbare Deviceherstellung.
3. Die Verbindungen zeigen bei Verwendung in OLEDs eine tiefblaue Emissionsfarbe.

Die Erfindung wird durch die nachfolgenden Beispiele näher erläutert, ohne sie dadurch einschränken zu wollen.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR (2-Brombiphenyl, Palladium(II)acetat, Tris-1-furylphosphin, Anorganika, Lösemittel) bezogen werden. Die Synthese von 10-Brom-5H-dibenzo[a,d]cyclohepten-5-on ist in der Literatur (B. Taljaard et al., Eur. J. Org. Chem. 2005, 12, 2607), die von 5H-Dibenzo[a,d]cyclohepten ist in der Literatur (C. Schmuck et al., Synthesis 2002, 5, 655), die von 10-Brom-dibenz[b,f]oxepin ist in der Literatur (M. Nogradi et al., Acta Chimica Academiae Scientiarum Hungaricae 1978, 96(4), 393) beschrieben.

### Beispiel 1: Tris(4-(10-dibenzo[a,d]cyclohepten-5-spiro-9-bifluoren)-phenyl)amin

### A) 10-Brom-dibenzo[a,d]cyclohepten-5-spiro-9-bifluoren

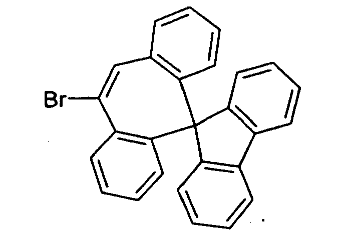

Aus einer Mischung von 49.0 ml (284 mmol) 2-Brombiphenyl und 4.3 ml (55 mmol) 1,2-Dichlorethan in 400 ml THF und 8.1 g (333 mmol) Magnesium wird das entsprechende Grignardreagenz dargestellt. Zu dieser Lösung lässt man unter gutem Rühren eine Lösung von 77.0 g (270 mmol) 10-Bromo-5H-dibenzo[a,d]cyclohepten-5-on in 400 ml THF schnell zulaufen und rührt 16 h bei Raumtemperatur nach. Man versetzt die Lösung mit 20 ml EtOH, entfernt das Lösemittel komplett im Vakuum, nimmt den Rückstand in 1000 ml Eisessig auf, versetzt mit 5 ml konz. Salzsäure, kocht die Mischung 3 h unter Rückfluss, versetzt dann mit 200 ml Toluol, lässt unter Rühren erkalten, saugt vom kristallinen Niederschlag ab, wäscht diesen dreimal mit je 100 ml Eisessig, dreimal mit 100 ml Ethanol und trocknet dann in Vakuum. Ausbeute: 102.0 g (89.6 % d. Th.), Reinheit ca. 98% ig n. NMR.

### B) Tris(4-(10-dibenzo[a,d]cyclohepten-5-spiro-9-bifluoren)phenyl)-amin

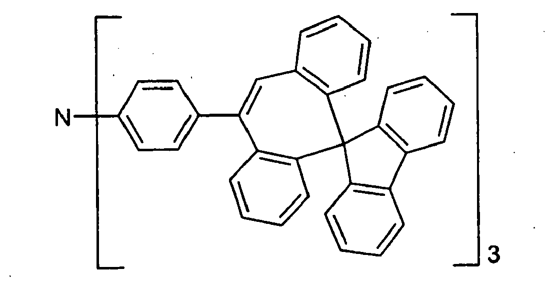

Eine auf -78°C gekühlte Lösung von 25.3 g (60 mmol) 10-Brom-5H-dibenzo[a,d]cyclohepten-5-spiro-9-bifluoren in 600 ml THF wird tropfenweise mit 26.0 ml (65 mmol) n-Butyllithium (2.5 M in n-Hexan) versetzt und 2 h bei -78 °C nachgerührt. Dann gibt man 8.0 ml (72 mmol) Borsäuretrimethylester zu, rührt 30 min. bei -78 °C nach, erwärmt im Warmwasserbad auf 0 °C, versetzt mit 600 ml Dioxan, 180 ml 1.0 M Natriumcarbonatlösung, 8.7 g (18 mmol) Tris(4-bromphenyl)amin, 135 mg (0.6 mmol) Palladium(II)acetat und 1.4 g (6 mmol) Tris-2-furylphosphin und erhitzt die Mischung 16 h unter Rückfluss. Nach Erkalten wird der Niederschlag abgesaugt, dreimal mit 200 ml Wasser und dreimal mit 100 ml Ethanol gewaschen, getrocknet, fünfmal aus Dioxan (ca. 40 ml/g) umkristallisiert und dann bei T = 365 °C, p = 5 x 10⁻⁵ mbar sublimiert. Ausbeute: 9.8 g (43.2 % d. Th.), Reinheit 99.9 %ig n. HPLC.

### Beispiel 2: Tris(4-(10-dibenzo[a,d]cyclohepten-5-spiro-9-(2,7-di-tert-butyl)bifluoren)phenyl)amin

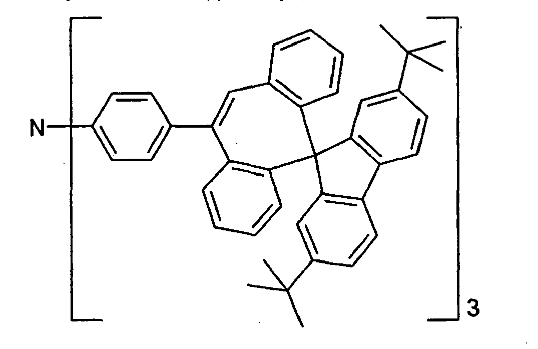

Durchführung analog Beispiel 1, wobei anstatt 2-Brombiphenyl 98.1 g (284 mmol) 2-Brom-4,4'-di-tert-butyl-biphenyl eingesetzt werden. Sublimation bei T = 360 °C, p = 5 x 10⁻⁵ mbar. Ausbeute: 16.4 g (56.7 % d. Th.), Reinheit 99.9 %ig n. HPLC.

### Beispiel 3: Diphenyl-(4-(10-dibenzo[a,d]cyclohepten-5-spiro-9-bifluoren)phenyl)amin

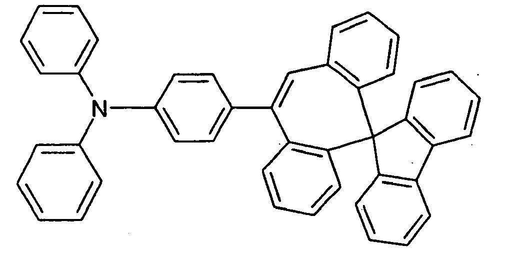

Durchführung analog Beispiel 1, wobei anstatt Tris(4-bromphenyl)amin 17.8 g (55 mmol) Diphenyl-(4-bromphenyl)amin eingesetzt werden. Umkristallisation aus Toluol / Acetonitril. Sublimation bei T = 315°C, p = 5 x 10⁻⁵ mbar. Ausbeute: 22.9 g (71.0 % d. Th.), Reinheit 99.9 %ig n. HPLC.

### Weitere Beispiele:

Analog Beispiel 3 werden folgende Verbindungen, ausgehend von den gezeigten Bromarylaminen, dargestellt.

| Beispiel | Boronsäure | Produkt | Ausbeute |
|---|---|---|---|
| 4 | 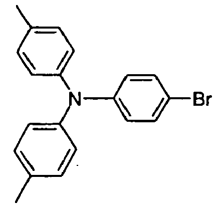 | 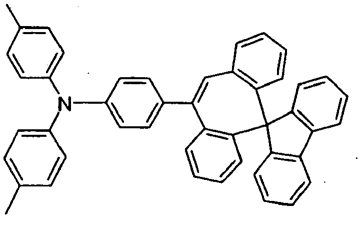 | 45.0% |
| 5 | 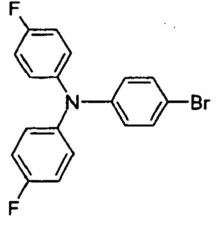 | 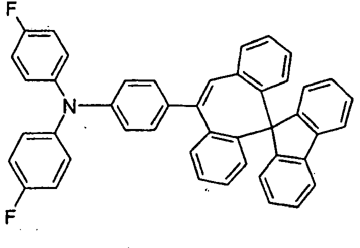 | 67.5 % |
| 6 | 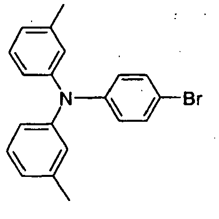 | 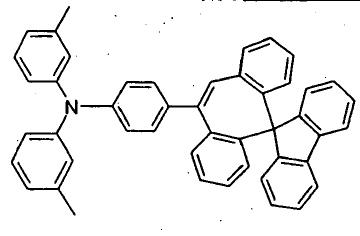 | 71.8% |
| 7 | 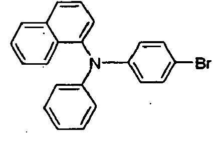 | 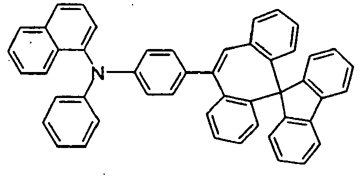 | 73.4% |
| 8 | 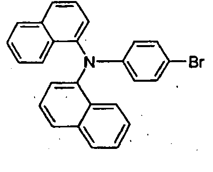 | 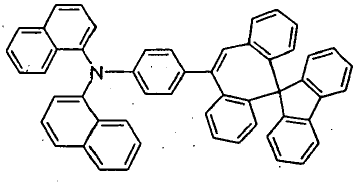 | 77.7% |
| 9 | 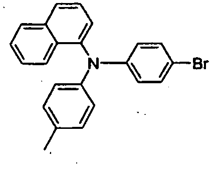 | 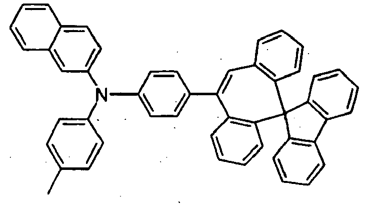 | 65.2% |

### Beispiel 10: : Tris(4-(10-(5-dimethyl)dibenzo[a,d]cyclohepten)-phenyl)-amin

### A) 5-Dimethyl-dibenzo[a,d]cyclohepten

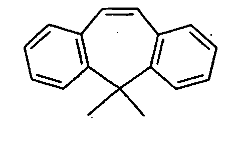

Eine Lösung von 19.2 g (100 mmol) 5H-Dibenzo[a,d]cyclohepten in 300 ml DMSO wird bei 10 °C mit 12.3 g (110 mmol) Kalium-tert-butylat versetzt, 10 min. gerührt, dann mit 7.2 ml (115 mmol) Methyliodid versetzt und 30 min. nachgerührt. Anschließend wird bei 10°C mit 12.3 g (110 mmol) Kalium-tert-butylat versetzt, 10 min. gerührt, dann mit 7.2 ml (115 mmol) Methyliodid versetzt und 30 min. nachgerührt. Man gibt 500 ml Wasser zu, saugt vom Feststoff ab und kristallisiert diesen dreimal aus Butanol um. Ausbeute: 14.8 g (67.1 % d. Th.), Reinheit 95 %ig n. NMR.

### B) 10-Brom-5-dimethyl-dibenzo[a,d]cyclohepten

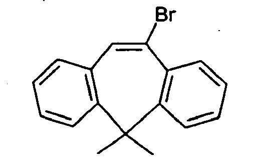

Eine Suspension von 110.2 g (500 mmol) 5-Dimethyl-dibenzo[a,d]cyclohepten in 1000 ml Eisessig wird tropfenweise mit 38.4 ml (750 mmol) Brom versetzt und 16 h bei Raumtemperatur gerührt. Der kristalline Feststoff wird abgesaugt, mit wenig Eisessig und Ethanol gewaschen und getrocknet.

Zu einer Lösung von 60 g (1.5 mol) Natriumhydroxid in 3000 ml Methanol bei 50 °C gibt man den so erhaltenen Feststoff unter gutem Rühren zu, kocht die Mischung 1.5 h unter Rückfluss, destilliert dann 1500 ml Methanol ab, gibt die gleiche Menge Wasser zu, lässt erkalten, saugt vom kristallinen Feststoff ab, wäscht diesen dreimal mit je 200 ml Wasser und dreimal mit je 200 ml Methanol und trocknet im Vakuum. Ausbeute: 132.4 g (88.5% d. Th.), Reinheit 98 %ig n. NMR.

### C) Tris(4-(10-(5-dimethyl)dibenzo[a,d]cyclohepten)-phenyl)amin

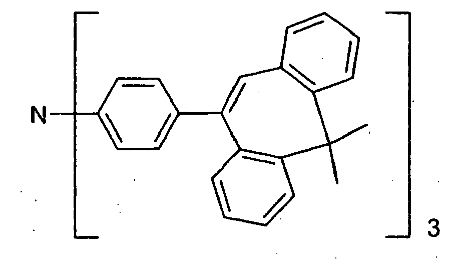

Durchführung analog Beispiel 1 B, wobei anstatt 10-Brom-5H-dibenzo[a,d]-cyclohepten-5-spiro-9-bifluoren 18.0 g (60 mmol) 10-Brom-5-dimethyldibenzo[a,d]cyclohepten eingesetzt werden. Umkristallisation aus DMF. Sublimation bei T = 320°C, p = 5 x 10⁻⁵ mbar. Ausbeute: 8.7 g (53.8 % d. Th.), Reinheit 99.9 %ig n. HPLC.

### Beispiel 11: Tris(4-(10-dibenzo[b,f]oxepin)-phenyl)amin

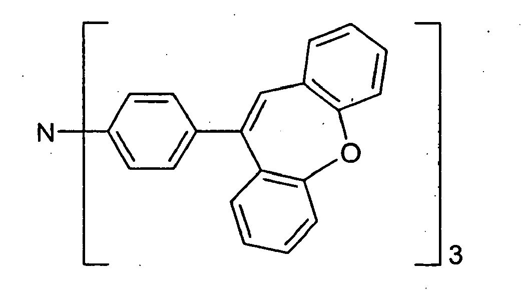

Durchführung analog Beispiel 1 B, wobei anstatt 10-Brom-5H-dibenzo[a,d]-cyclohepten-5-spiro-9-bifluoren 16.4 g (60 mmol) 10-Brom-dibenzo[b,f]-oxepin eingesetzt werden. Umkristallisation aus NMP. Sublimation bei T = 310 °C, p = 5 x 10⁻⁵ mbar. Ausbeute: 6.9 g (46.5 % d. Th.), Reinheit 99.9 %ig n. HPLC.

### Beispiel 12: Vergleich der thermischen Stabilität

Zum Vergleich der thermischen Stabilität der erfindungsgemäßen Verbindungen nach Beispiel 1, 2, 3, 10 und 11 mit der offenkettigen Styrylverbindung Tris(4-stilben)amin [114869-94-2] gemäß dem Stand der Technik wurden jeweils 100 mg dieser Verbindungen mit einer Reinheit von 99.9 % n. HPLC unter Vakuum in Ampullen abgeschmolzen und dann 100 h bei 280 °C ausgelagert.

Die HPLC-MS Analyse ergibt eine sehr gute thermische Stabilität der erfindungsgemäßen Verbindungen nach Beispiel 1, 2, 3, 10 und 11, die keine wesentliche Veränderung zeigen. Dagegen unterliegt die Vergleichsverbindung Tris(4-stilben)amin weitgehender Zersetzung; unter den beschriebenen Bedingungen sind nach 100 h nur noch ca. 25 % Tris(4-stilben)amin nachweisbar. Neben oligomeren Anteilen bildet sich hauptsächlich 4,4'-Di-tert-butylstilben.

### Beispiel 13: Herstellung der OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 14 bis 21 werden die Ergebnisse verschiedener OLEDs vorgestellt. Glasplatten, die mit strukturiertem ITO (Indium Zinn Oxid) beschichtet sind, bilden die Substrate der OLEDs. Zur verbesserten Prozessierung wird PEDOT (aus Wasser aufgeschleudert; bezogen von H. C. Starck, Goslar, Deutschland; Poly(3,4-ethylendioxy-2,5-thiophen)) direkt auf das Substrat aufgebracht. Die OLEDs bestehen immer aus folgender Schichtenfolge: Substrat / PEDOT 20 nm / Lochinjektionsschicht (HIL1) 20 nm / Lochtransportschicht (HTM1) 20 nm / Emissionsschicht (EML) 30 nm / Elektronentransportschicht (ETM1) 20 nm und abschließend eine Kathode. Die Materialien bis auf PEDOT werden in einer Vakuumkammer thermisch aufgedampft. Dabei besteht die EML immer aus einem Matrixmaterial (Host) und einem Dotierstoff (Guest oder Dotand), der durch Coverdampfung dem Host beigemischt wird. Als Hostmaterial wird der Host H1 verwendet. Die Kathode wird durch eine 1 nm dünne LiF-Schicht und eine darauf abgeschiedene 150 nm Al-Schicht gebildet. Die Tabelle 1 zeigt die chemischen Strukturen der zum Aufbau der OLEDs verwendeten Materialien.

Diese OLEDs werden standardmäßig charakterisiert; hierfür werden die Elektrolumineszenzspektren, die Effizienz (gemessen in cd/A), die Leistungseffizienz (gemessen in Im/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 1000 cd/m² auf die Hälfte gesunken ist.

In Tabelle 2 sind die Ergebnisse einiger OLEDs (Beispiele 14 bis 21) zusammengefasst.

**Tabelle 1 - Verwendete Materialien**

| | | |
|---|---|---|
| 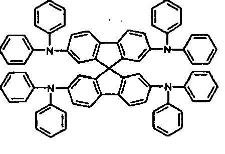 | 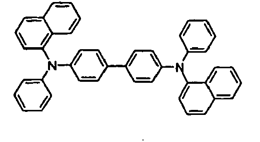 | 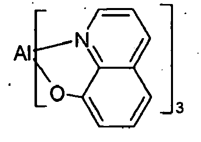 |
| HIL1 | HTM1 | ETM1 |
| 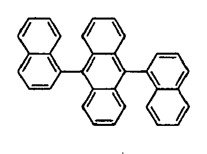 | 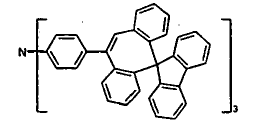 | 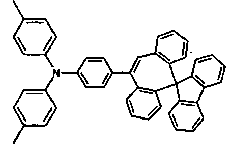 |
| H1 | D1 (Beispiel 1) | D4 (Beispiel 4) |
| 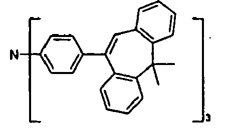 | 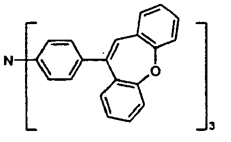 | |
| D10 (Beispiel 10) | D11 (Beispiel 11) | |

**Tabelle 2 - Ergebnisse einiger OLEDs**

| **Bsp.** | **EML** | **Max. Effizienz (cd/A)** | **Spannung (V) bei 1000 cd/m²** | **CIE** | **Lebensdauer (h) bei 1000 cd/m²** |
|---|---|---|---|---|---|
| 14 | H1 + 5% D1 | 6.5 | 5.6 | x=0.17 / y=0.19 | 2200 |
| 15 | H1+3% D1 | 6.3 | 5.7 | x=0.16 / y=0.18 | 1800 |
| 16 | H1+5% D4 | 10.5 | 5.5 | x=0.18 / y=0.21 | 2400 |
| 17 | H1 + 3% D4 | 3.8 | 5.8 | x=0.18 / y=0.20 | 2000 |
| 18 | H1 + 5% D10 | 6.2 | 5.5 | x=0.17 / y=0.18 | 2300 |
| 19 | H1 + 3% D10 | 6.1 | 5.7 | x=0.16 / y=0.18 | 2100 |
| 20 | H1 + 5% D11 | 12.5 | 5.4 | x=0.18 / y=0.45 | 8100 |
| 21 | H1 + 7% D11 | 13.5 | 5.3 | x=0.18 / y=0.46 | 8500 |

## Patentansprüche

1. Verbindungen gemäß Formel (1), 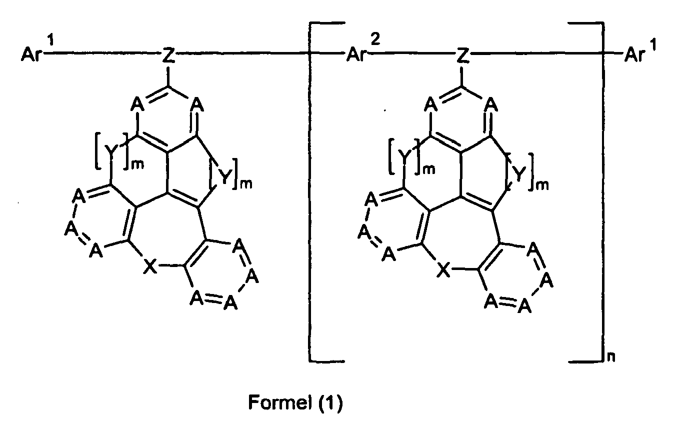 wobei für die verwendeten Symbole und Indizes gilt:
Z ist gleich oder verschieden bei jedem Auftreten N, P, As oder P=O;
X, Y ist gleich oder verschieden bei jedem Auftreten CR₂, C=O, O, S, NR, SiR₂, PR, P(=O)R, S(=O) oder SO₂;
A ist gleich oder verschieden bei jedem Auftreten CR oder N;
Ar¹ ist gleich oder verschieden bei jedem Auftreten ein monovalentes aromatisches oder heteroaromatisches Ringsystem mit 5 bis 40 aromatischen Ringatomen, welches mit einem oder mehreren Resten R substituiert sind kann;
Ar² ist gleich oder verschieden bei jedem Auftreten ein bivalentes aromatisches oder heteroaromatisches Ringsystem mit 5 bis 40 aromatischen Ringatomen, welches mit einem oder mehreren Resten R substituiert sind kann;
R ist gleich oder verschieden bei jedem Auftreten H, F, Cl, Br, I, CN, NO₂, Si(R¹)₃, N(R¹)₂, B(OR¹)₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R¹ substituiert sein kann, wobei eine oder mehrere nicht benachbarte CH₂-Gruppen durch -R¹C=CR¹-, -C≡C-, Si(R¹)₂, Ge(R¹)₂, Sn(R¹)₂, C=O, C=S, C=Se, C=NR¹, -O-, -S-, -N(R¹)- oder -CONR¹- ersetzt sein können und wobei ein oder mehrere H- Atome durch F, Cl, Br, I, CN oder NO₂ ersetzt sein können, oder ein aromatisches oder heteroaromatisches Ringsystem mit 5 bis 40 aromatischen Ringatomen, das mit einem oder mehreren Resten R¹ substituiert sein kann, oder eine Aryloxy- oder Heteroaryloxygruppe mit 5 bis 24 aromatischen Ringatomen, die durch einen oder mehrere Reste R¹ substituiert sein kann, oder eine Kombination aus zwei, drei, vier oder fünf dieser Systeme; dabei können auch zwei oder mehrere Substituenten R miteinander ein mono- oder polycyclisches, aliphatisches oder aromatisches Ringsystem bilden;
R¹ ist bei jedem Auftreten gleich oder verschieden H oder ein Kohlenwasserstoffrest mit 1 bis 20 C-Atomen, welcher aliphatisch oder aromatisch oder eine Kombination aus aliphatisch und aromatisch sein kann; dabei können auch ein oder mehrere H- Atome durch F ersetzt sein; dabei können auch zwei oder mehrere Reste R² miteinander ein mono- oder polycyclisches, aliphatisches oder aromatisches Ringsystem bilden;
n ist 0, 1, 2 oder 3;
m ist gleich oder verschieden bei jedem Auftreten 0 oder 1, wobei m = 0 bedeutet, dass die Gruppe Y nicht vorhanden ist und stattdessen in diesen Positionen Reste R gebunden sind.

2. Verbindungen nach Anspruch 1, **dadurch gekennzeichnet, dass** das Symbol Z, gleich oder verschieden bei jedem Auftreten, für N oder P=O steht.

3. Verbindungen nach Anspruch 1 oder 2, **dadurch gekennzeichnet, dass** maximal ein Symbol A in jedem aromatischen Ring für N steht und die anderen Symbole A in diesem aromatischen Ring für CR stehen.

4. Verbindungen nach einem oder mehreren der Ansprüche 1 bis 3, **dadurch gekennzeichnet, dass** mindestens eine Gruppe Ar¹ für eine Gruppe der Formel (2) steht, 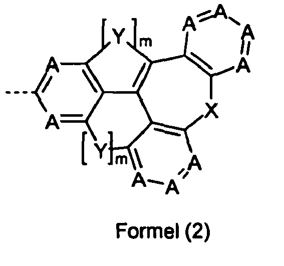 wobei die Symbole und Indizes dieselbe Bedeutung haben, wie in Anspruch 1 beschrieben, und die gestrichelte Bindung die Anknüpfung an Z bedeutet.

5. Verbindungen nach einem oder mehreren der Ansprüche 1 bis 4, **dadurch gekennzeichnet, dass** die Verbindung gemäß Formel (1) für eine Verbindung der Formel (1a) steht, 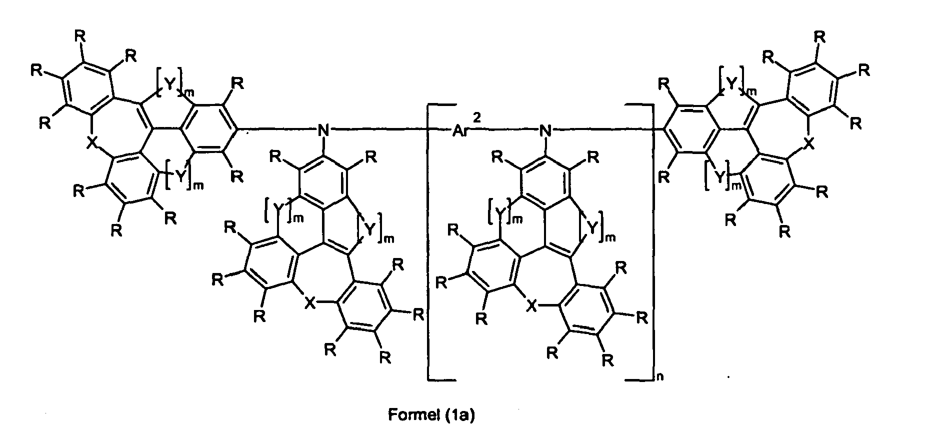 wobei die Symbole und Indizes dieselbe Bedeutung haben, wie in Anspruch 1 beschrieben.

6. Verbindungen nach einem oder mehreren der Ansprüche 1 bis 5, **dadurch gekennzeichnet, dass** das Symbol X, gleich oder verschieden bei jedem Auftreten, für CR₂, O, NR oder SiR₂ steht.

7. Verbindungen nach einem oder mehreren der Ansprüche 1 bis 6, **dadurch gekennzeichnet, dass**, wenn das Symbol X für eine Gruppe der Formel CR₂ steht, das Symbol R an dieser Gruppe X, gleich oder verschieden bei jedem Auftreten, bevorzugt für eine geradkettige Alkylgruppe mit 1 bis 4 C-Atomen oder eine verzweigte Alkylgruppe mit 3 oder 4 C-Atomen, wobei jeweils ein oder mehrere H-Atome durch F ersetzt sein können, oder für eine Aryl- oder Heteroarylgruppe mit 5 bis 10 aromatischen Ringatomen, oder eine Kombination aus zwei oder drei dieser Systeme steht; dabei können auch die zwei Reste R der Gruppe CR₂ miteinander ein mono- oder polycyclisches, aliphatisches oder aromatisches Ringsystem bilden und **dadurch** ein Spirosystem aufbauen.

8. Verbindungen nach einem oder mehreren der Ansprüche 1 bis 7 gemäß Formel (1 b), 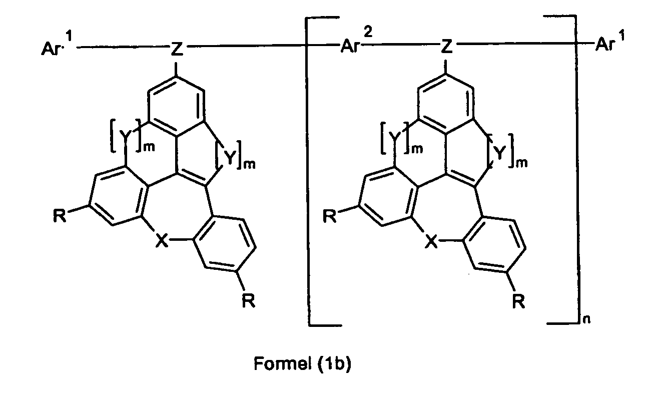 wobei die Symbole und Indizes dieselbe Bedeutung haben, wie in Anspruch 1 beschrieben.

9. Verbindungen nach einem oder mehreren der Ansprüche 1 bis 8, **dadurch gekennzeichnet, dass** sie eine dreizählige Symmetrieachse aufweisen.

10. Polymere, Oligomere oder Dendrimere enthaltend eine oder mehrere Verbindungen nach einem oder mehreren der Ansprüche 1 bis 9, wobei ein oder mehrere Reste R Bindungen der Verbindung zum Polymer, Oligomer oder Dendrimer darstellen.

11. Verfahren zur Herstellung von Verbindungen nach einem oder mehreren der Ansprüche 1 bis 9, **dadurch gekennzeichnet, dass** ein Boronsäurederivat gemäß Formel (4), 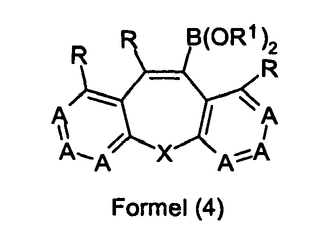 wobei die Symbole die in Anspruch 1 aufgeführte Bedeutung haben, in einer Suzuki-Kupplung mit der zentralen Einheit der Verbindung gemäß Formel (1) umgesetzt wird, wobei diese Chlor, Brom, Iod, Triflat, Tosylat oder OSO₂R¹ als reaktive Gruppe enthält, wobei R¹ dieselbe Bedeutung hat, wie in Anspruch 1 beschrieben.

12. Verwendung von Verbindungen nach einem oder mehreren der Ansprüche 1 bis 10 in organischen elektronischen Vorrichtungen.

13. Organische elektronische Vorrichtung, insbesondere organische Elektrolumineszenzvorrichtungen (OLEDs, PLEDs), organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs), organische Photorezeptoren, lichtemittierende elektrochemische Zellen (LECs) und organische Laserdioden (O-Laser), enthaltend mindestens eine organische Schicht; **dadurch gekennzeichnet, dass** die organische Schicht mindestens eine Verbindung nach einem oder mehreren der Ansprüche 1 bis 10 enthält.

14. Organische Elektrolumineszenzvorrichtung nach Anspruch 13, **dadurch gekennzeichnet, dass** die Verbindung nach einem oder mehreren der Ansprüche 1 bis 10 als emittierende Verbindung in einer emittierenden Schicht verwendet wird, bevorzugt in Kombination mit einem Hostmaterial, und/oder als Lochtransport- bzw. Lochinjektionsmaterial, bevorzugt in einer Lochtransport- bzw. Lochinjektionsschicht, und/oder als Elektronentransportmaterial und/oder als Lochblockiermaterial für fluoreszierende oder phosphoreszierende OLEDs und/oder als Triplett-Matrixmaterial für phosphoreszierende OLEDs.

15. Mischung enthaltend mindestens eine Verbindung nach einem oder mehreren der Ansprüche 1 bis 10 und mindestens ein Hostmaterial.

## Claims

1. Compounds of the formula (1), 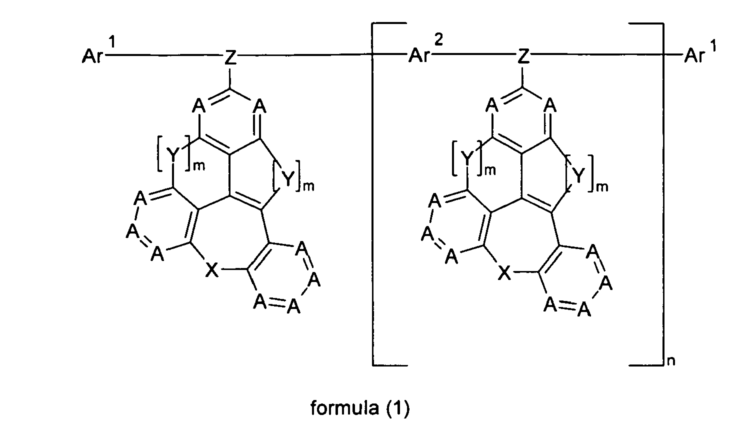 where the following applies to the symbols and indices used:
Z is, identically or differently on each occurrence, N, P, As or P=O;
X, Y are, identically or differently on each occurrence, CR₂, C=O, O, S, NR, SiR₂, PR, P(=O)R, S(=O) or SO₂;
A is, identically or differently on each occurrence, CR or N;
Ar¹ is, identically or differently on each occurrence, a monovalent aromatic or heteroaromatic ring system having 5 to 40 aromatic ring atoms, which may be substituted by one or more radicals R;
Ar² is, identically or differently on each occurrence, a divalent aro- matic or heteroaromatic ring system having 5 to 40 aromatic ring atoms, which may be substituted by one or more radicals R;
R is, identically or differently on each occurrence, H, F, Cl, Br, I, CN, NO₂, Si(R¹)₃, N(R¹)₂, B(OR¹)₂, a straight-chain alkyl, alkoxy or thioalkoxy group having 1 to 40 C atoms or a branched or cyclic alkyl, alkoxy or thioalkoxy group having 3 to 40 C atoms, each of which may be substituted by one or more radicals R¹, where one or more non-adjacent CH₂ groups may be replaced by -R¹C=CR¹-, -C≡C-, Si(R¹)₂, Ge(R¹)₂, Sn(R¹)₂, C=O, C=S, C=Se, C=NR¹, -O-, -S-, -N(R¹)- or -CONR¹- and where one or more H atoms may be replaced by F, Cl, Br, I, CN or NO₂, or an aromatic or heteroaromatic ring system having 5 to 40 aromatic ring atoms, which may be substituted by one or more radicals R¹, or an aryloxy or heteroaryloxy group having 5 to 24 aromatic ring atoms, which may be substituted by one or more radicals R¹, or a combination of two, three, four or five of these systems; two or more substituents R here may also form a mono- or poly- cyclic, aliphatic or aromatic ring system with one another;
R¹ is on each occurrence, identically or differently, H or a hydro- carbon radical having 1 to 20 C atoms, which may be aliphatic or aromatic or a combination of aliphatic and aromatic; one or more H atoms here may also be replaced by F; two or more radicals R¹ here may also form a mono- or polycyclic, aliphatic or aromatic ring system with one another;
n is 0, 1, 2 or 3;
m is, identically or differently on each occurrence, 0 or 1, where m = 0 means that the group Y is not present and instead radi- cals R are bonded in these positions.

2. Compounds according to Claim 1, **characterised in that** the symbol Z, identically or differently on each occurrence, stands for N or P=O.

3. Compounds according to Claim 1 or 2, **characterised in that** a maximum of one symbol A in each aromatic ring stands for N and the other symbols A in this aromatic ring stand for CR.

4. Compounds according to one or more of Claims 1 to 3, **characterised in that** at least one group Ar¹ stands for a group of the formula (2), 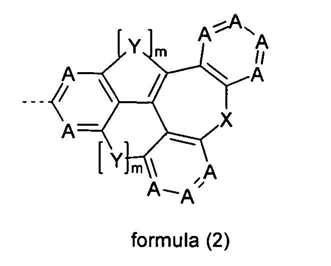 where the symbols and indices have the same meaning as described in Claim 1, and the dashed bond denotes the link to Z.

5. Compounds according to one or more of Claims 1 to 4, **characterised in that** the compound of the formula (1) stands for a compound of the formula (1a), 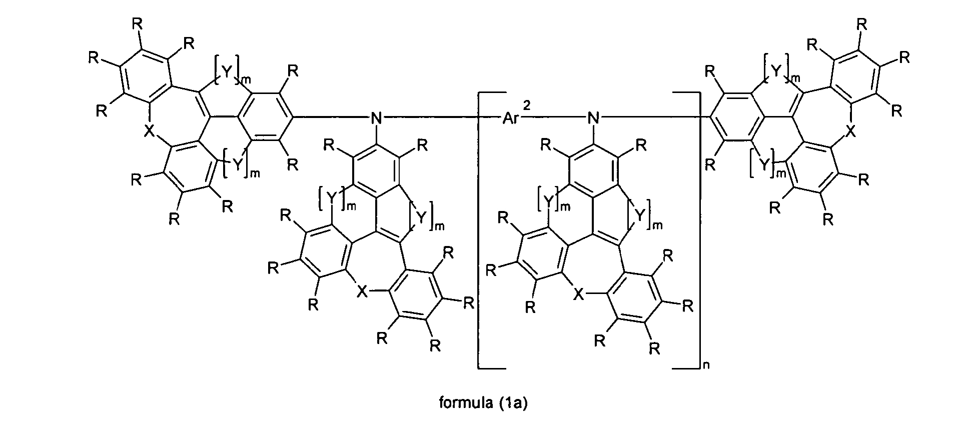 where the symbols and indices have the same meaning as described in Claim 1.

6. Compounds according to one or more of Claims 1 to 5, **characterised in that** the symbol X, identically or differently on each occurrence, stands for CR₂, O, NR or SiR₂.

7. Compounds according to one or more of Claims 1 to 6, **characterised in that**, if the symbol X stands for a group of the formula CR₂, the symbol R on this group X preferably stands, identically or differently on each occurrence, for a straight-chain alkyl group having 1 to 4 C atoms or a branched alkyl group having 3 or 4 C atoms, where in each case one or more H atoms may be replaced by F, or for an aryl or heteroaryl group having 5 to 10 aromatic ring atoms, or a combination of two or three of these systems; the two radicals R in the group CR₂ here may also form a mono- or polycyclic, aliphatic or aromatic ring system with one another and thus construct a spiro system.

8. Compounds according to one or more of Claims 1 to 7 of the formula (1b). 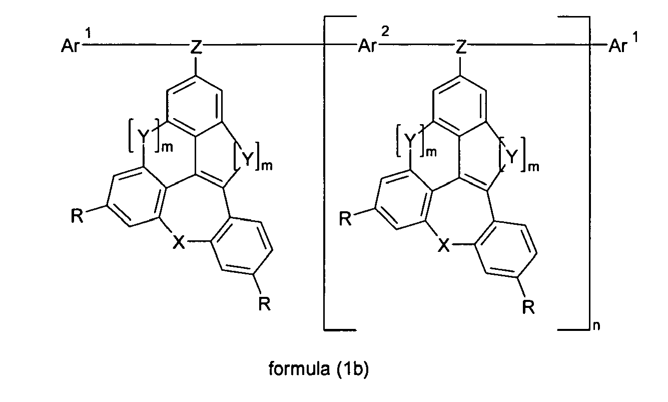 where the symbols and indices have the same meaning as described in Claim 1.

9. Compounds according to one or more of Claims 1 to 8, **characterised in that** they have a three-fold axis of symmetry.

10. Polymers, oligomers or dendrimers comprising one or more compounds according to one or more of Claims 1 to 9, where one or more radicals R represent bonds from the compound to the polymer, oligomer or dendrimer.

11. Process for the preparation of compounds according to one or more of Claims 1 to 9, **characterised in that** a boronic acid derivative of the formula (4), 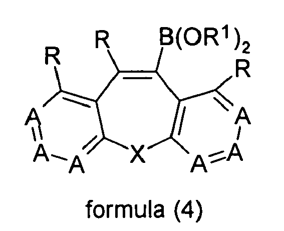 where the symbols have the meaning given in Claim 1, is reacted in a Suzuki coupling with the central unit of the compound of the formula (1) which contains chlorine, bromine, iodine, triflate, tosylate or OSO₂R¹ as reactive group, where R¹ has the same meaning as described in Claim 1.

12. Use of compounds according to one or more of Claims 1 to 10 in organic electronic devices.

13. Organic electronic device, in particular organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), organic photoreceptors, light-emitting electrochemical cells (LECs) and organic laser diodes (O-lasers), comprising at least one organic layer, **characterised in that** the organic layer comprises at least one compound according to one or more of Claims 1 to 10.

14. Organic electroluminescent device according to Claim 13, **characterised in that** the compound according to one or more of Claims 1 to 10 is used as emitting compound in an emitting layer, preferably in combination with a host material, and/or as hole-transport or hole-injection material, preferably in a hole-transport or hole-injection layer, and/or as electron-transport material and/or as hole-blocking material for fluorescent or phosphorescent OLEDs and/or as triplet matrix material for phosphorescent OLEDs.

15. Mixture comprising at least one compound according to one or more of Claims 1 to 10 and at least one host material.

## Revendications

1. Composés de la formule (1), 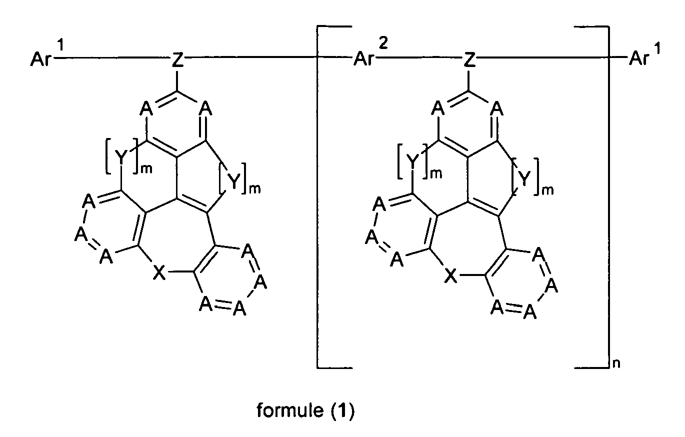 dans laquelle ce qui suit s'applique aux symboles et indices utilisés :
Z est, de manière identique ou différente pour chaque occurrence, N, P, As ou P=O ;
X, Y sont, de manière identique ou différente pour chaque occur- rence, CR₂, C=O, O, S, NR, SiR₂, PR, P(=O)R, S(=O) ou SO₂;
A est, de manière identique ou différente pour chaque occurrence, CR ou N ;
Ar¹ est, de manière identique ou différente pour chaque occurrence, un système de cycle aromatique ou hétéroaromatique mono- valent comportant de 5 à 40 atomes de cycle aromatique, lequel peut être substitué par un ou plusieurs radicaux R ;
Ar² est, de manière identique ou différente pour chaque occurrence, un système de cycle aromatique ou hétéroaromatique divalent comportant de 5 à 40 atomes de cycle aromatique, lequel peut être substitué par un ou plusieurs radicaux R ;
R est, de manière identique ou différente pour chaque occurrence, H, F, Cl, Br, l, CN, NO₂, Si(R¹)₃, N(R¹)₂, B(OR¹)₂, un groupe alkyle, alcoxy ou thioalcoxy en chaîne droite comportant de 1 à 40 atomes de C ou un groupe alkyle, alcoxy ou thioalcoxy rami- fié ou cyclique comportant de 3 à 40 atomes de C, dont chacun peut être substitué par un ou plusieurs radicaux R¹, où un ou plusieurs groupes CH₂ non adjacents peut/peuvent être rem- placé(s) par -R¹C=CR¹-, -C≡C-, Si(R¹)₂, Ge(R¹)₂, Sn(R¹)₂, C=O, C=S, C=Se, C=NR¹, -O-, -S-, -N(R¹)- ou -CONR¹- et où un ou plusieurs atomes de H peut/peuvent être remplacé(s) par F, Cl, Br, l, CN ou NO₂, ou un système de cycle aromatique ou hété- roaromatique comportant de 5 à 40 atomes de cycle aroma- tique, lequel peut être substitué par un ou plusieurs radicaux R¹, ou un groupe aryloxy ou hétéroaryloxy comportant de 5 à 24 atomes de cycle aromatique, lequel peut être substitué par un ou plusieurs radicaux R¹, ou une combinaison de deux, trois, quatre ou cinq de ces systèmes ; deux substituants R ou plus peuvent également former ici un système de cycle aliphatique ou aromatique mono- ou polycyclique l'un avec l'autre/les uns avec les autres ;
R¹ est, pour chaque occurrence, de manière identique ou diffé- rente, H ou un radical hydrocarbone comportant de 1 à 20 atomes de C, lequel peut être aliphatique ou aromatique ou une combinaison aliphatique et aromatique ; un ou plusieurs atomes de H peut/peuvent également être remplacé(s) ici par F ; deux radicaux R¹ ou plus peuvent également former ici un système de cycle aliphatique ou aromatique mono- ou polycyclique l'un avec l'autre/les uns avec les autres ;
n est 0, 1, 2 ou 3 ;
m est, de manière identique ou différente pour chaque occurrence, 0 ou 1, où m = 0 signifie que le groupe Y n'est pas présent et qu'en lieu et place, des radicaux R sont liés au niveau de ces positions.

2. Composés selon la revendication 1, **caractérisés en ce que** le symbole Z, de manière identique ou différente pour chaque occurrence, représente N ou P=O.

3. Composés selon la revendication 1 ou 2, **caractérisés en ce qu'**un maximum d'un symbole A dans chaque cycle aromatique représente N et les autres symboles A dans ce cycle aromatique représentent CR.

4. Composés selon une ou plusieurs des revendications 1 à 3, **caractérisés en ce qu'**au moins un groupe Ar¹ représente un groupe de la formule (2), 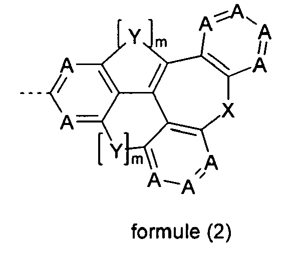 dans laquelle les symboles et les indices présentent la même signification que décrit dans la revendication 1, et la liaison en pointillés représente le lien sur Z.

5. Composés selon une ou plusieurs des revendications 1 à 4, **caractérisés en ce que** le composé de la formule (1) représente un composé de la formule (1a), 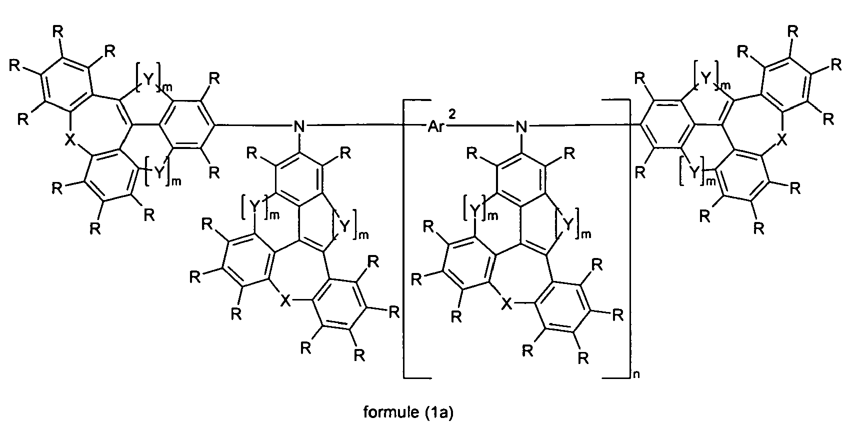 dans laquelle les symboles et les indices présentent la même signification que décrit dans la revendication 1.

6. Composés selon une ou plusieurs des revendications 1 à 5, **caractérisés en ce que** le symbole X, de manière identique ou différente pour chaque occurrence, représente CR₂, O, NR ou SiR₂.

7. Composés selon une ou plusieurs des revendications 1 à 6, **caractérisés en ce que**, si le symbole X représente un groupe de la formule CR₂, le symbole R sur ce groupe X représente de préférence, de manière identique ou différente pour chaque occurrence, un groupe alkyle en chaîne droite comportant de 1 à 4 atomes de C ou un groupe alkyle ramifié comportant 3 ou 4 atomes de C, où, dans chaque cas, un ou plusieurs atomes de H peut/peuvent être remplacé(s) par F, ou un groupe aryle ou hétéroaryle comportant de 5 à 10 atomes de cycle aromatique, ou une combinaison de deux ou trois de ces systèmes ; les deux radicaux R dans le groupe CR₂ peuvent également former ici un système de cycle aliphatique ou aromatique mono- ou polycyclique l'un avec l'autre et par conséquent construire un système spiro.

8. Composés selon une ou plusieurs des revendications 1 à 7 de la formule (1 b), 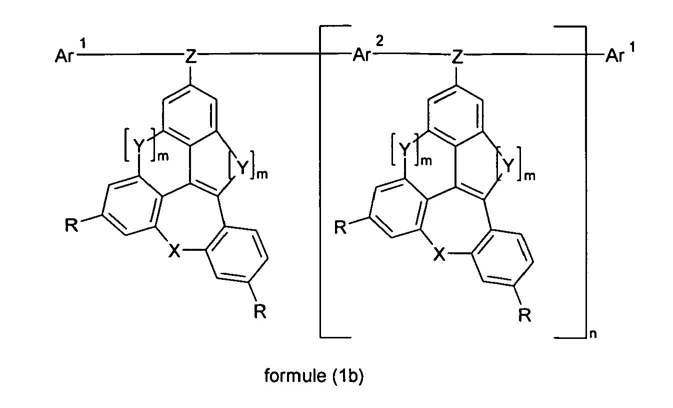 dans laquelle les symboles et les indices présentent la même signification que décrit dans la revendication 1.

9. Composés selon une ou plusieurs des revendications 1 à 8, **caractérisés en ce qu'**ils présentent un axe de symétrie triple.

10. Polymères, oligomères ou dendrimères comprenant un ou plusieurs composés selon une ou plusieurs des revendications 1 à 9, où un ou plusieurs radicaux R représente/représentent des liaisons depuis le composé sur le polymère, l'oligomère ou le dendrimère.

11. Procédé pour la préparation de composés selon une ou plusieurs des revendications 1 à 9, **caractérisé en ce qu'**un dérivé d'acide boronique de la formule (4), 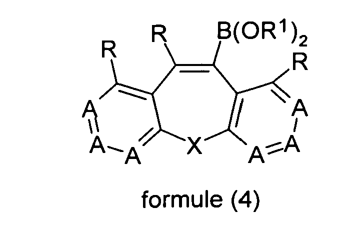 dans laquelle les symboles présentent la signification donnée dans la revendication 1, est amené à réagir dans un couplage de Suzuki avec l'unité centrale du composé de la formule (1), laquelle contient chlore, brome, iode, triflate, tosylate ou OSO₂R¹ en tant que groupe réactif, où R¹ présente la même signification que décrit dans la revendication 1.

12. Utilisation de composés selon une ou plusieurs des revendications 1 à 10 dans des dispositifs électroniques organiques.

13. Dispositif électronique organique, en particulier dispositifs électroluminescents organiques (OLED, PLED), transistors à effet de champ organiques (O-FET), transistors à film mince organiques (O-TFT), transistors émetteurs de lumière organiques (O-LET), circuits intégrés organiques (O-IC), cellules solaires organiques (O-SC), dispositifs à extinction de champ organiques (O-FQD), photorécepteurs organiques, cellules électrochimiques émettrices de lumière (LEC) et diodes laser organiques (O-lasers), comprenant au moins une couche organique, **caractérisé en ce que** la couche organique comprend au moins un composé selon une ou plusieurs des revendications 1 à 10.

14. Dispositif électroluminescent organique selon la revendication 13, **caractérisé en ce que** le composé selon une ou plusieurs des revendications 1 à 10 est utilisé en tant que composé émetteur dans une couche émettrice, de préférence en combinaison avec un matériau hôte, et/ou en tant que matériau de transport de trous ou matériau d'injection de trous, de préférence dans une couche de transport de trous ou une couche d'injection de trous, et/ou en tant que matériau de transport d'électrons et/ou en tant que matériau de blocage de trous pour des OLED fluorescentes ou phosphorescentes et/ou en tant que matériau de matrice triplet pour des OLED phosphorescentes.

15. Mélange comprenant au moins un composé selon une ou plusieurs des revendications 1 à 10 et au moins un matériau hôte.
